# EUROPEAN PATENT SPECIFICATION

(11) **EP 4 585 196 B1**
(45) Date of publication and mention of the grant of the patent: **07.10.2026**
(21) Application number: 23894555.4
(22) Date of filing: 20.11.2023
(51) Int. Cl.: A61F 13/49, A61F 13/496, B32B 5/02, B32B 5/26

(54) **STRETCHABLE SHEET AND UNDERPANTS-TYPE ABSORBENT ARTICLE**
DEHNBARE FOLIE UND SAUGFÄHIGER ARTIKEL VOM UNTERHOSEN-TYP
FEUILLE ÉTIRABLE ET ARTICLE ABSORBANT DE TYPE CULOTTE

(30) Priority: 21.11.2022 JP 2022186028
(43) Date of publication of application: 16.07.2025
(73) Proprietor: Unicharm Corporation, Ehime 799-0111 (JP)
(72) Inventor: TANAKA, Suguru, Kanonji-shi, Kagawa 769-1602 (JP); SHIMAZU, Takeshi, Kanonji-shi, Kagawa 769-1602 (JP); MUKAI, Hirotomo, Kanonji-shi, Kagawa 769-1602 (JP); SHIMADA, Yusei, Kanonji-shi, Kagawa 769-1602 (JP)
(74) Representative: Dolleymores
(86) International application number: PCT/JP2023/041623
(87) International publication number: WO 2024/111548

(56) References cited:
- WO-A1-2018/154684
- WO-A1-2019/069806
- WO-A1-2019/092967
- JP-A- 2019 170 731
- JP-A- 2020 054 744
- JP-A- 2020 516 409
- US-A1- 2019 374 404
- US-A1- 2020 155 371

## Description

### FIELD

The present invention relates to a stretchy sheet and an underpants-shaped absorbent article.

### BACKGROUND

As an example of a sheet in an absorbent article such as a disposable diaper, a stretchy sheet has been conventionally known in which elastic members such as elastic strings in a state of being stretched are sandwiched between two sheets and such two sheets are joined together. For example, Patent Document 1 discloses a stretchy sheet in which elastic members are sandwiched between two sheets constituted by fiber sheets, and in which such two sheets are joined by a plurality of fused portions that are located on two sides of each elastic member and provided at intervals along an extending direction of the elastic members. Patent Documents 2 and 3 disclose absorbent articles and Patent Document 4 discloses a manufacturing method of a stretchable sheet.

### [CITATION LIST]

### [PATENT LITERATURE]

Patent Document 1: Japanese Patent Application Publication No. 2020-199246
Patent Document 2: US Patent Application Publication No. US2019/374404
Patent Document 3: US Patent Application Publication No. US2020/15537
Patent Document 4: Japanese Patent Application Publication No. 2020-054744

### SUMMARY

### [TECHNICAL PROBLEM]

As in Patent Document 1, in the case where a plurality of fused portions are provided intermittently along the extending direction of the elastic members, aesthetic wrinkles can be formed along a direction orthogonal to the extending direction of the elastic members (along a product-longitudinal direction) to improve the texture and aesthetics. However, such a formation of the aesthetic wrinkles causes rising of the wrinkles, which may increase the frictional force. Furthermore, such wrinkles are less likely to be compressed, which may cause discomfort during usage. In the stretchy sheet of Patent Document 1, a part of a region where one of the two sheets is overlapped with the elastic members as viewed in the thickness direction has a region where the porosity between constituent fibers is relatively high, thereby reducing the degree to which the elastic members press against the wearer's skin. However, there is a need for improvement in flexibility even in regions other than the region where the sheet is overlapped with the elastic members in the thickness direction.

The present invention has been made in view of the circumstances described above, and its object is to provide a stretchy sheet in which aesthetic wrinkles extending along the vertical direction of the stretchy sheet are formed while improving flexibility to reduce discomfort during usage.

### [SOLUTION TO PROBLEM]

A main aspect of the present invention for achieving the above-described aspect is a stretchy sheet having a vertical direction and a lateral direction that intersect each other, the stretchy sheet including: a plurality of elastic members that are located between a pair or sheets joined by a plurality of joining portions, the plurality of elastic members being arranged at intervals in the vertical direction, the plurality of elastic members being capable of stretching and contracting in the lateral direction; and a joining-portion group that includes at least one pair of the joining portions sandwiching each of the elastic members, a plurality of the joining-portion groups constituting a first joining-portion row, a second joining-portion row, and a third joining-portion row that are arranged in the vertical direction, in a state where the stretchy sheet is stretched in the lateral direction, in each of the joining-portion groups, a vertical length between the joining portions that are arranged adjacently in the vertical direction being shorter than a lateral length of each of the joining portions, a non-arrangement region being located between the joining-portion groups that are arranged adjacently in the vertical direction, the non-arrangement region being a region where the joining-portion groups are not arranged, a vertical length of the non-arrangement region being longer than the vertical length between the pair of the joining portions, the first joining-portion row, the second joining-portion row, and the third joining-portion row being arranged adjacently in the lateral direction, in a certain position in the vertical direction, one of the joining portions in the first joining-portion row, one of the joining portions in the second joining-portion row, and one of the joining portions in the third joining-portion row being overlapped with each other, in another certain position in the vertical direction, the non-arrangement region in the first joining-portion row, the non-arrangement region in the second joining-portion row, and the non-arrangement region in the third joining-portion row being overlapped with each other.

Features of the present invention other than the above will become clear by reading the description of the present specification with reference to the accompanying drawings.

### [ADVANTAGEOUS EFFECT OF THE INVENTION]

According to the present invention, it is possible to provide a stretchy sheet in which aesthetic wrinkles extending along the vertical direction of the stretchy sheet are formed while improving flexibility to reduce discomfort during usage.

### BRIEF DESCRIPTION OF DRAWINGS

FIG. 1 is a schematic perspective view of an underpants-shaped disposal diaper 1.
FIG. 2 is a schematic plan view of the diaper 1 in an unfolded and stretched state, as viewed from a wearer's skin side.
FIG. 3 is a schematic cross-sectional view taken along a line A-A of FIG. 2.
FIG. 4 is a diagram illustrating joining-portion rows R provided in a front waist portion 20.
FIGS. 5A and 5B are diagrams each illustrating the function of attaching a waist elastic member 23.
FIG. 6 is an enlarged view of some of the joining-portion rows R.
FIG. 7 is an enlarged view of parts of the joining-portion rows R of FIG. 6.
FIG. 8 is a diagram illustrating the joining-portion rows R in an upper end portion 20A shown in FIG. 4.
FIG. 9 is a diagram illustrating regions between joining-portion groups 50G provided adjacently in the lateral direction.
FIG. 10 is a diagram illustrating an arrangement of joining-portion rows R according to a modified example.
FIG. 11 is a diagram illustrating joining-portion rows R according to a modified example.
FIG. 12 is a diagram illustrating joining-portion rows R according to another modified example.
FIG. 13 is a diagram illustrating adhesion portions 70 in a folded portion 22r.

### DESCRIPTION OF EMBODIMENTS

At least the following matters will become clear with the description of this specification and the attached drawings. Aspect 1 is a stretchy sheet having a vertical direction and a lateral direction that intersect each other, the stretchy sheet including: a plurality of elastic members that are located between a pair or sheets joined by a plurality of joining portions, the plurality of elastic members being arranged at intervals in the vertical direction, the plurality of elastic members being capable of stretching and contracting in the lateral direction; and a joining-portion group that includes at least one pair of the joining portions sandwiching each of the elastic members, a plurality of the joining-portion groups constituting a first joining-portion row, a second joining-portion row, and a third joining-portion row that are arranged in the vertical direction, in a state where the stretchy sheet is stretched in the lateral direction, in each of the joining-portion groups, a vertical length between the joining portions that are arranged adjacently in the vertical direction being shorter than a lateral length of each of the joining portions, a non-arrangement region being located between the joining-portion groups that are arranged adjacently in the vertical direction, the non-arrangement region being a region where the joining-portion groups are not arranged, a vertical length of the non-arrangement region being longer than the vertical length between the pair of the joining portions, the first joining-portion row, the second joining-portion row, and the third joining-portion row being arranged adjacently in the lateral direction, in a certain position in the vertical direction, one of the joining portions in the first joining-portion row, one of the joining portions in the second joining-portion row, and one of the joining portions in the third joining-portion row being overlapped with each other, in another certain position in the vertical direction, the non-arrangement region in the first joining-portion row, the non-arrangement region in the second joining-portion row, and the non-arrangement region in the third joining-portion row being overlapped with each other.

According to the stretchy sheet of Aspect 1, the joining portion in the first joining-portion row, the joining portion in the second joining-portion row, and the joining portion in the third joining-portion row that are arranged adjacently in the lateral direction are provided at a common position in the vertical direction, thereby making the aesthetic winkles along the vertical direction likely to be formed. In addition, the non-arrangement region where the joining-portion groups are not arranged is a region likely to be compressed, and the non-arrangement regions in the first joining-portion row, the second joining-portion row, and the third joining-portion row that are arranged adjacently in the lateral direction are located at a common position in the vertical direction. As a result, a space along the lateral direction is formed to be easily compressed. The above-described configuration improves flexibility of the stretchy sheet to reduce the possibility of discomfort during usage.

Aspect 2 is the stretchy sheet according to Aspect 1, wherein, in a state where the stretchy sheet is stretched in the lateral direction, a vertical length of each of the joining-portion groups is longer than the lateral length of each of the joining-portion groups.

According to the stretchy sheet of Aspect 2, the joining-portion groups are each long in the vertical direction, thereby being easier to form the wrinkles extending along the vertical direction.

Aspect 3 is the stretchy sheet according to Aspect 1 or 2, wherein, in a state where the stretchy sheet is stretched in the lateral direction, out of the non-arrangement region in the first joining-portion row, the non-arrangement region in the second joining-portion row, and the non-arrangement region in the third joining-portion row that are overlapped with each other in the other certain position, two non-arrangement regions include: an overlap region where the two non-arrangement regions are overlapped with each other with respect to the vertical direction; and a non-overlap region where the two non-arrangement regions are not overlapped with each other with respect to the vertical direction, and a vertical length of the overlap region is longer than a vertical length of the non-overlap region.

According to the stretchy sheet of Aspect 3, the overlap region is a region where a space along the lateral direction is likely to be formed. The vertical length of the overlap region is long, thereby making a larger space be easily secured. This can improve flexibility.

Aspect 4 is the stretchy sheet according to any one of Aspects 1 to 3, wherein, in a state where the stretchy sheet is stretched in the lateral direction, the vertical length of the non-arrangement region is longer than the vertical length of each of the joining-portion groups.

According to the stretchy sheet of Aspect 4, the length of the non-arrangement region where the joining-portion groups are not arranged is long, which can ensure the space that is likely to be compressed.

Aspect 5 is the stretchy sheet according to Aspect 4, wherein, the vertical length of the non-arrangement region is three times or less than the vertical length of each of the joining-portion groups.

According to the stretchy sheet of Aspect 5, in the case where the non-arrangement region without the joining-portion groups is excessively long, the winkles along the vertical direction are less likely to be formed. However, the vertical length of the non-arrangement region is set to be three times or less than the vertical length of the joining-portion group to prevent the vertical distance between the joining-portion groups from being excessively long. This promotes formation of the wrinkles extending along the vertical direction using the joining-portion group.

Aspect 6 is the stretchy sheet according to any one of Aspects 1 to 3, wherein, in a state where the stretchy sheet is stretched in the lateral direction, the vertical length of the non-arrangement region is shorter than the vertical length of each of the joining-portion groups.

According to the stretchy sheet of Aspect 6, the vertical length of each of the joining-portion groups is longer than the vertical length of the non-arrangement region, thereby being easier to form the wrinkles extending along the vertical direction.

Aspect 7 is the stretchy sheet according to any one of Aspects 1 to 6, wherein, in a state where the stretchy sheet is stretched in the lateral direction, an inter-row non-arrangement region is located between the joining-portion groups that are arranged adjacently in the lateral direction, the inter-row non-arrangement region being a region where the joining-portion groups are not arranged, and a lateral length of the inter-row non-arrangement region is longer than the vertical length of each of the joining-portion groups.

According to the stretchy sheet of Aspect 7, in the case where the lateral length of the inter-row non-arrangement region is short, the wrinkles extending along the vertical direction are likely to be formed finely. This may result in hard texture. The lateral length of the inter-row non-arrangement region is long, thereby making large wrinkles extending along the vertical direction likely to be formed. This can improve the texture.

Aspect 8 is the stretchy sheet according to Aspect 7, wherein, the lateral length of the inter-row non-arrangement region is three times or less than the vertical length of each of the joining-portion groups.

According to the stretchy sheet of Aspect 8, in the case where the lateral length of the inter-row non-arrangement region where the joining-portion groups are not provided is excessively long, the aesthetic winkles are less likely to be formed along the vertical direction. Thus, the lateral length of the inter-row non-arrangement region is three times or less than the vertical length of each joining-portion group, so that the distance between the joining-portion groups arranged adjacently in the lateral direction can be prevented from being excessively large. This promotes the formation of the wrinkles extending along the vertical direction using the joining-portion groups.

Aspect 9 is the stretchy sheet according to any one of Aspects 1 to 8, wherein, an auxiliary joining portion that joins the pair of sheets together is provided in a part of the non-arrangement region, and the auxiliary joining portion being different from the joining portions.

According to the stretchy sheet of Aspect 8, in the case of a long distance between the joining-portion groups that are arranged adjacently in the vertical direction (i.e., the vertical length of each non-arrangement region), the regular wrinkles extending along the vertical direction may be unlikely to be formed. In such a case, the auxiliary joining portion is provided in a part of the non-arrangement region, which makes regular wrinkles extending along the vertical direction likely to be formed.

Aspect 10 is the stretchy sheet according to any one of Aspects 1 to 9, wherein, in a state where the elastic members contract, the non-arrangement region in the first joining-portion row, the non-arrangement region in the second joining-portion row, and the non-arrangement region in the third joining-portion row that are overlapped with each other in the other position in the vertical direction each have a protrusion, the protrusion protruding toward one side or another side of the stretchy sheet in a thickness direction of the stretchy sheet.

According to the stretchy sheet of Aspect 10, the joining portions are not arranged in each of the non-arrangement regions. Thus, each of the non-arrangement regions is a region likely to be compressed. At a time of contraction of the elastic members, the wrinkles along the vertical direction on two laterally-outward sides of each joining-portion group extend on two laterally-outward sides of each of the non-arrangement regions. In each of the non-arrangement regions that is likely to be compressed, without the joining portions, the protrusion protruding toward one side or another side of the stretchy sheet in the thickness direction thereof due to contraction. Such a plurality of protrusions having comfortable texture are formed to improve flexibility of the stretchy sheet.

Aspect 11 is the stretchy sheet according to any one of Aspects 1 to 10, wherein each of the joining groups includes: an upper-end joining portion referring to the joining portion on an upper vertical end of each of the joining-portion groups; a lower-end joining portion referring to the joining portion on a lower vertical end of each of the joining-portion groups, and a vertical length of the upper-end joining portion and a vertical length of the lower-end joining portion are each longer than a vertical length of each of the joining portions other than the upper-end joining portion and the lower-end joining portion in each of the joining-portion groups.

According to the stretchy sheet of Aspect 11, the elastic member is arranged between any one pair of the joining portions in each joining-portion group. However, in such a case, in the manufacturing process, when the elastic member is attempted to be arranged between the pair of joining portions including the upper-end joining portion or the lower-end joining portion, the elastic member may be arranged at a shifted position. In each joining-portion group, the vertical lengths of the upper-end joining portion and the lower-end joining portion increase, and thus, the elastic member that is arranged at an excessively shifted position can be cut by the upper-end joining portion or the lower-end joining portion. This can avoid the arrangement of the elastic member in an unexpected position.

Aspect 12 is an underpants-shaped absorbent article having a vertical direction, a lateral direction, and a front-back direction that intersect each other, the underpants-shaped absorbent article including: a stretchy sheet according to any Aspects 1 to 11; an absorbent main body; and a pair of waist portions, the stretchy sheet being provided in at least one of the pair of waist portions, the vertical direction of the stretchy sheet extending along the vertical direction of the underpants-shaped absorbent article, the lateral direction of the stretchy sheet extending along the lateral direction of the underpants-shaped absorbent article, a thickness direction of the stretchy sheet extending along the front-back direction of the underpants-shaped absorbent article, the pair of waist portions being joined together by a pair of side joining portions, on two lateral sides of the pair of waist portions, at least one of the pair of waist portions including a stacked portion or a folded portion, the stacked portion being a portion where the pair of sheets adheres to another sheet other than the pair of sheets via an adhesion portion such that the pair of sheets and the other sheet are overlapped with each other with respect to the front-back direction, the folded portion being a portion where at least one of the pair of sheets is folded toward an inner surface of the at least one of the pair of waist portions and adheres to the inner surface of the at least one of the waist portions via the adhesion portion, the underpants-shaped absorbent article including a portion where the non-arrangement region is overlapped with a non-adhesion portion other than the adhesion portion in the stacked portion or a non-adhesion portion other than the adhesion portion in the folded portion, in a plan view.

According to the stretchy sheet of Aspect 12, the portion where the non-arrangement region is overlapped with the non-adhesion portion in the stacked portion or the non-adhesion portion in the folded portion is a region where the adhesion portions for adhesion of materials are not present while increasing the number of stacked materials in the thickness direction. Such a region is thus likely to be compressed. Such compressible regions can improve the texture.

### Embodiment

Hereinafter, an embodiment of an underpants-shaped absorbent article including a stretchy sheet will be described by way of example of an underpants-shaped disposable diaper for adults.

### Basic Configuration of underpants-shaped disposable diaper 1

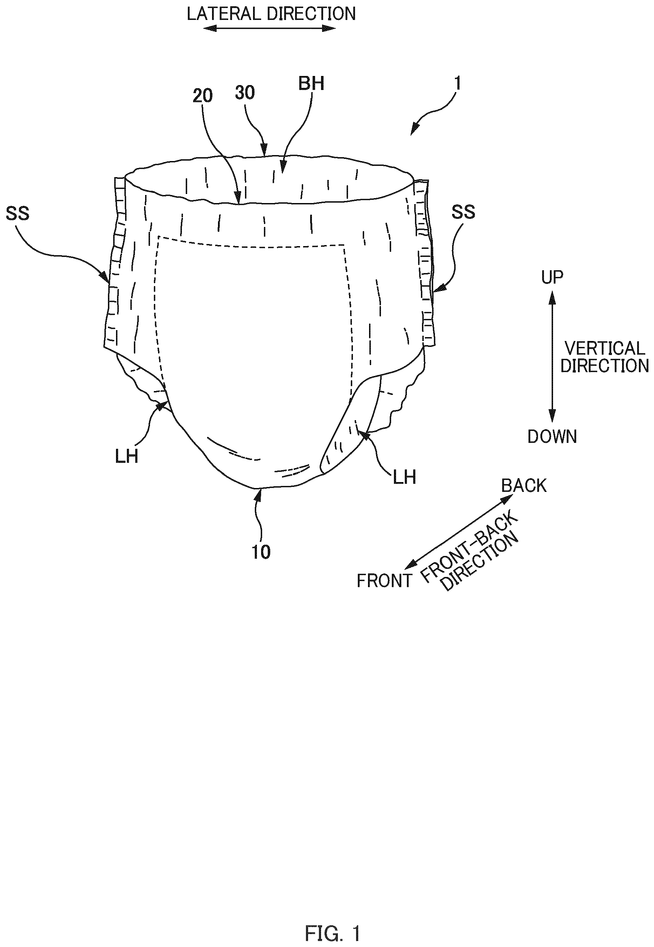

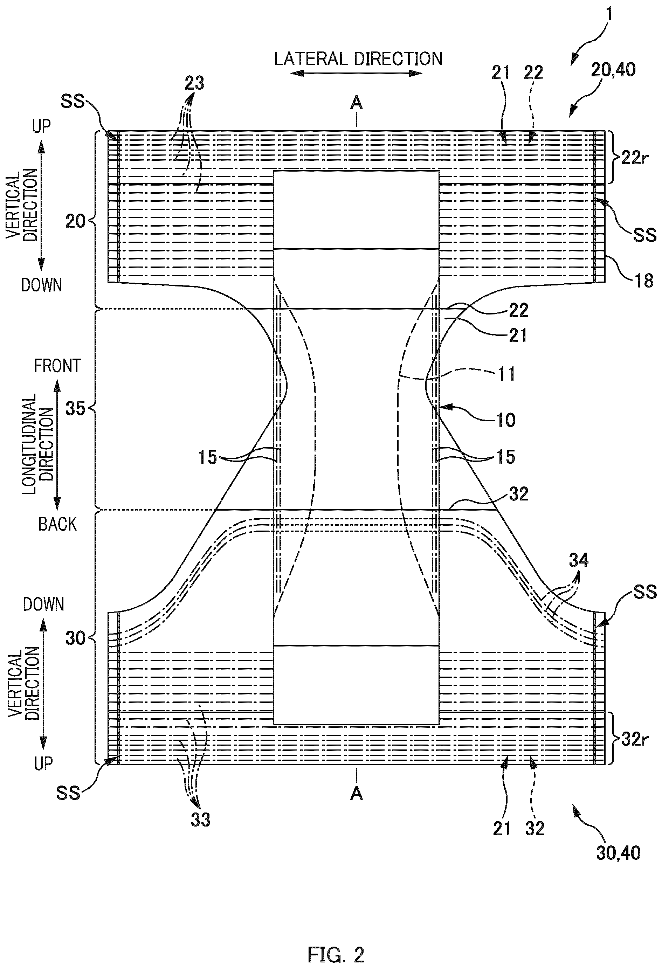

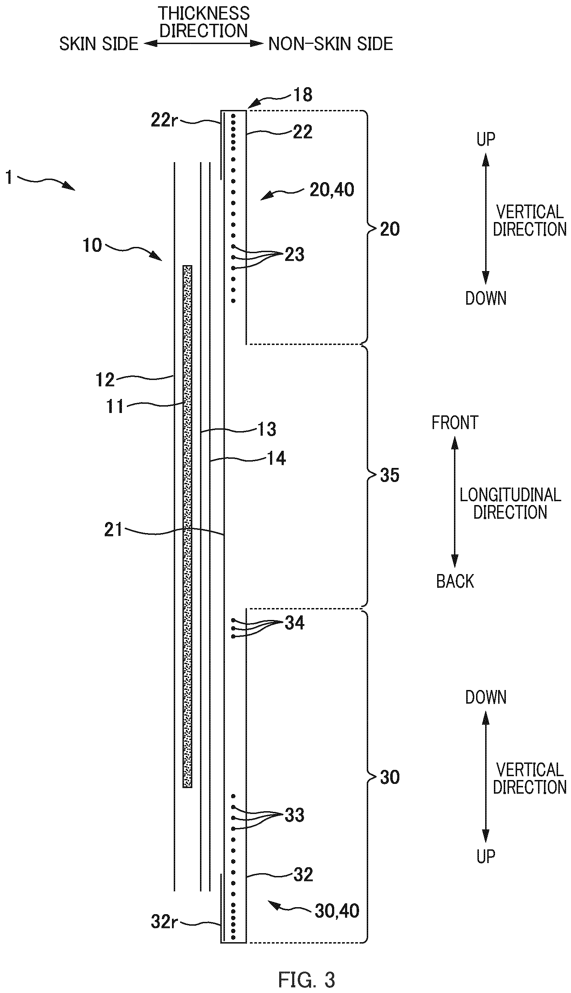

FIG. 1 illustrates a schematic perspective view of an underpants-shaped disposal diaper 1 (hereinafter also referred to as a "diaper"). FIG. 2 illustrates a schematic plan view of the diaper 1 in an unfolded and stretched state, as viewed from a wearer's skin side. FIG. 3 illustrates a schematic cross-sectional view taken along a line A-A of FIG. 2.

The "unfolded state" of the diaper 1 refers to a state where side joining portions SS (which will be described later) on two lateral sides of the diaper 1 in an underpants-shaped state shown in FIG. 1 are detached from each other such that a front waist portion 20 (which will be described later) and a back waist portion 30 (which will be described later) separate from each other and the diaper 1 is opened in the vertical direction, and thus the diaper 1 is unfolded in a plan view. In addition, the "stretched state" of the diaper 1 refers to a state in which a product (diaper 1) is stretched without wrinkles, specifically, a state where the diaper 1 is stretched until the dimensions of constituent members of the diaper 1 (for example, an absorbent main body 10, the front waist portion 20, or the like) match or are close to the dimensions of the members on their own.

In the underpants-shaped state while the diaper 1 is put on shown in FIG. 1, the diaper 1 has a vertical direction, a lateral direction, and a front-back direction that intersect each other, and includes a waist opening BH and a pair of leg openings LH. In the vertical direction, the waist opening BH side is the upper side, and the wearer's crotch side is the lower side. In the front-back direction, the wearer's waist side is the front side, and the wearer's back side is the back side. In addition, in the unfolded and stretched state shown in FIG. 2, the diaper 1 has a longitudinal direction, a lateral direction, and a thickness direction as three directions orthogonal to one another (i.e., a direction passing through the paper surface in FIG. 2). The longitudinal direction extends along the above-mentioned vertical direction. One side in the longitudinal direction corresponds to the front side, and the other side corresponds to the back side. In addition, the diaper 1 has a thickness direction in which materials are overlaid on each other as shown in FIG. 3, and in the thickness direction, the side that comes into contact with the wearer refers to a skin side, and the side opposite to the skin side refers to a non-skin side.

The diaper 1 includes the absorbent main body 10 and an exterior body 18. The exterior body 18 includes a pair of waist portions 20, 30, and a crotch portion 35 that is located at the wearer's crotch. The exterior body 18 is joined to the non-skin-side surface of the absorbent main body 10 using an adhesive or the like. Out of the pair of waist portions 20, 30, the one on the front side is referred to as the front waist portion 20, and the one on the back side is referred to as the back waist portion 30. The front waist portion 20 and the back waist portion 30 are joined together on two lateral sides thereof, by a pair of side joining portions SS. Examples of the joining method using the side joining portions SS include thermal welding, ultrasonic welding, and joining using an adhesive.

As shown in FIG. 3, the absorbent main body 10 includes an absorbent core 11, a liquid-permeable top sheet 12 arranged on the skin side with respect to the absorbent core 11, a liquid-impermeable back sheet 13 arranged on the non-skin side with respect to the absorbent core 11, and an exterior sheet 14. The absorbent core 11 only needs to absorb and hold an excreted fluid, and examples thereof include an absorbent core that is obtained by forming the liquid absorbent material such as pulp fibers and superabsorbent polymer into a predetermined shape. The absorbent core 11 may be covered with a liquid-permeable sheet such as tissue paper, nonwoven fabric sheet, or the like.

As shown in FIG. 2, on two lateral sides of the absorbent main body 10, leg elastic members 15 (e.g., elastic strings) may be provided, which stretches and contracts along the longitudinal direction of the absorbent main body 10. In addition, leak-proof-wall portions capable of standing up toward the skin side may be formed by, for example, elastic members that stretch and contract in the longitudinal direction being arranged inside the leg elastic members 15 in the lateral direction.

As shown in FIGS. 2 and 3, the exterior body 18 has an exterior top sheet 21, a front-side exterior back sheet 22, and a back-side exterior back sheet 32.

The exterior top sheet 21 is arranged from the front waist portion 20 to the back waist portion 30 through the crotch portion 35 (FIG. 3). The width (lateral length) of the exterior top sheet 21 is larger in the front waist portion 20 and the back waist portion 30 than in the crotch portion 35 (FIG. 2). The exterior top sheet 21 can be formed of an air-through nonwoven fabric sheet, a spunbond nonwoven fabric sheet, an SMS nonwoven fabric sheet (spunbond/meltblown/spunbond nonwoven fabric sheet), a waterproof film, or the like.

As shown in FIGS. 2 and 3, the front-side exterior back sheet 22 is provided on the non-skin side with respect to the exterior top sheet 21, and the front-side exterior back sheet 22 and the exterior top sheet 21 constitute the front waist portion 20. The longitudinal outward end (upper end) of the front-side exterior back sheet 22 is folded toward the skin side. Such a folded portion 22r of the front-side exterior back sheet 22 wraps the longitudinal one end (outward end) of the exterior top sheet 21. The longitudinal outward end (upper end) of the front-side exterior back sheet 22 need not be folded.

In the same manner, as shown in FIGS. 2 and 3, the back-side exterior back sheet 32 is provided on the non-skin side with respect to the exterior top sheet 21, and the back-side exterior back sheet 32 and the exterior top sheet 21 constitute the back waist portion 30. The longitudinal outward end (upper end) of the back-side exterior back sheet 32 is folded toward the skin side. Such a folded portion 32r of the back-side exterior back sheet 32 wraps the longitudinal other end (outward end) of the exterior top sheet 21. The longitudinal outward end (upper end) of the back-side exterior back sheet 32 need not be folded.

The front-side exterior back sheet 22 and the back-side exterior back sheet 32 can be formed of an air-through nonwoven fabric sheet, a spunbond nonwoven fabric sheet, an SMS nonwoven fabric sheet (spunbond/meltblown/spunbond nonwoven fabric), or the like.

The front waist portion 20 and the back waist portion 30 have the waist elastic members 23, 33 (e.g., elastic strings), respectively, that stretch and contract in the lateral direction. The plurality of waist elastic members 23, 33 are arranged at intervals in the vertical direction, and positioned between the exterior top sheet 21 and the front-side exterior back sheet 22, and between the exterior top sheet 21 and the back-side exterior back sheet 32 (see FIGS. 2 and 3).

In the present embodiment, in the back waist portion 30, back leg elastic members 34 are provided between the exterior top sheet 21 and the back-side exterior back sheet 32. For example, the back leg elastic members 34 may be a plurality of elastic strings or a band-shaped elastic sheet having stretchability, or the like. The back leg elastic members 34 are joined and fixed in a stretched state, using an adhesive such as a hot-melt adhesive.

The basic configuration of the diaper 1 has been described above, but the above-described configuration of the diaper 1 is an example, and there is no limitation to that configuration. For example, in the present embodiment, the diaper 1 includes the exterior body 18 having three portions: the front waist portion 20; the back waist portion 30; and the crotch portion 35 that connects the front waist portion 20 and the back waist portion 30. However, the configuration is not limited thereto. For example, in the diaper, the front waist portion 20 and the back waist portion 30 may be separate members such that the non-skin-side surface of the absorbent main body 10 is exposed at the crotch portion. Alternatively, the diaper may include an exterior member as a single member that extends continuously from the front waist portion 20 to the back waist portion 30.

### Waist Portions 20, 30 (Stretchy Sheet 40)

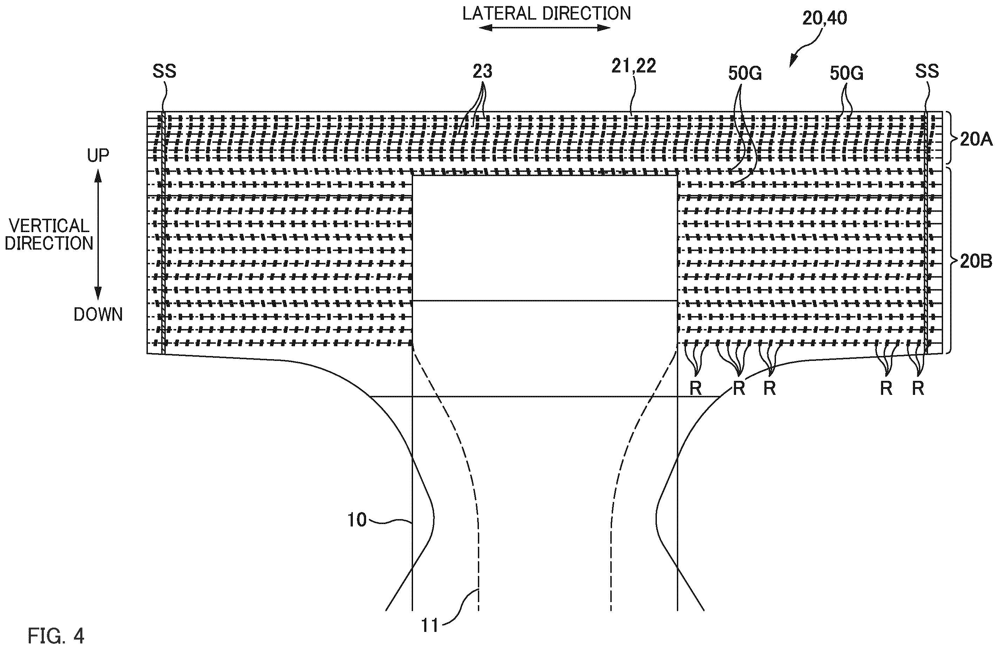

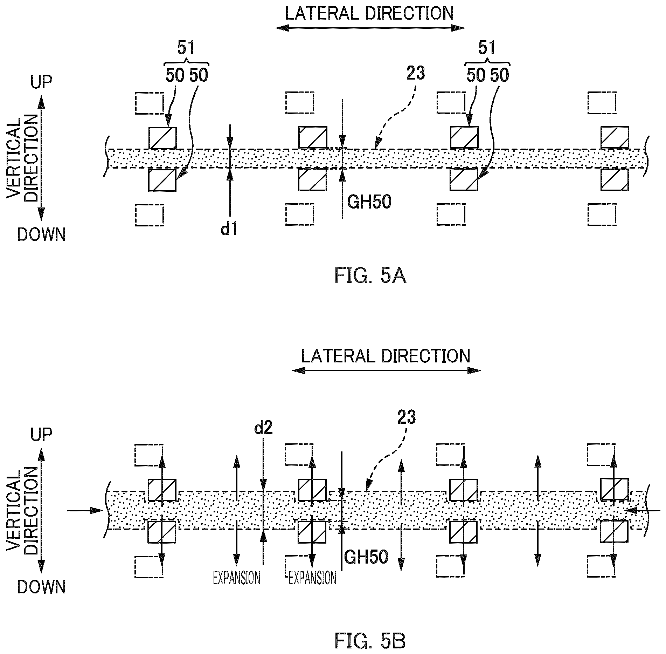

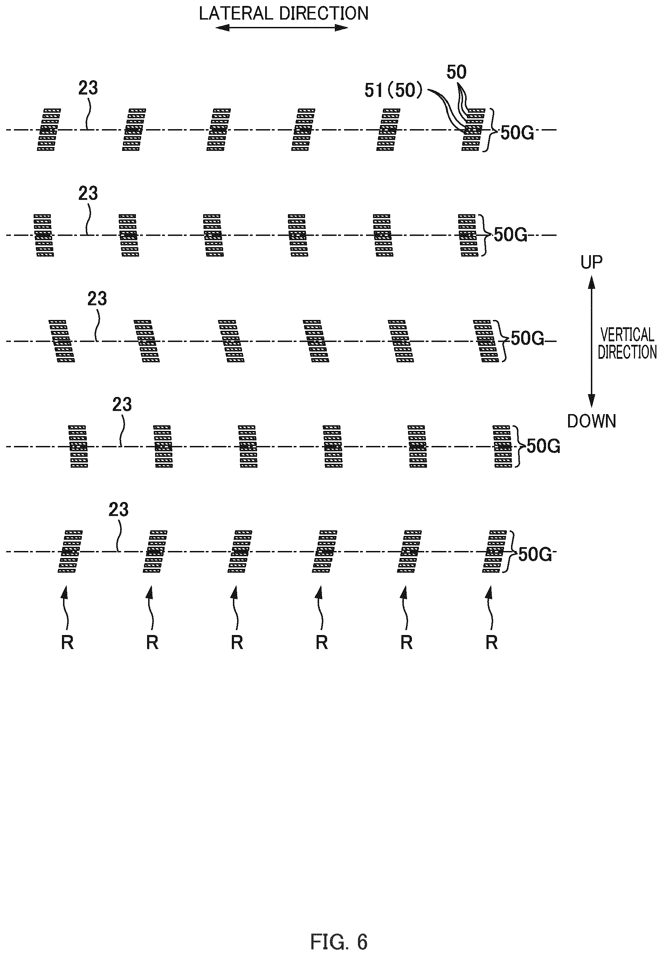

FIG. 4 is a diagram illustrating joining-portion rows R provided in the front waist portion 20. FIGS. 5A and 5B are diagrams each illustrating the function of attaching the waist elastic member 23. FIG. 6 is an enlarged view of some of the joining-portion rows R.

FIG. 4 and FIG. 6 each illustrate a state in which the front waist portion 20 (stretchy sheet 40) is stretched in the lateral direction. The state in which the waist portions 20, 30 and the stretchy sheet 40 have been stretched refers to a state in which the waist portions 20, 30 and the stretchy sheet 40 have been stretched to the extent that wrinkles formed in the waist portions 20, 30 and the stretchy sheet 40 can substantially no longer be seen, or in other words, a state in which the waist portions 20, 30 and the stretchy sheet 40 are stretched such that the dimensions of the constituent members of the waist portions 20, 30 and the stretchy sheet 40 (e.g., the exterior top sheet 21, and the like) match or are close to the dimensions of the members on their own.

### Position Restriction of Waist Elastic Members 23

The front waist portion 20 and the back waist portion 30 are constituted by: the exterior top sheet 21 and the front-side exterior back sheet 22 that are joined together by a plurality of joining portions 50; or the stretchy sheet 40 in which the waist elastic members 23, 33 (elastic members) capable of stretching and contracting in the lateral direction are arranged between the exterior top sheet 21 and the back-side exterior back sheet 32 (between a pair of sheets) at intervals in the vertical direction. As shown in FIGS. 4 and 6, the exterior top sheet 21 and the front-side exterior back sheet 22 (back-side exterior back sheet 32) are intermittently joined by the plurality of joining portions 50 that are discretely arranged in the vertical direction and in the lateral direction. The joining portions 50 can be formed using a known welding method such as ultrasonic welding, heat welding, or the like.

The pair of sheets joined by the joining portions 50 may be two separate sheets as with the exterior top sheet 21 and the front-side exterior back sheet 22 (back-side exterior back sheet 32) of the present embodiment, or may be a single sheet that has been folded to form two layers. The joining portions 50 join not only the pair of sheets that sandwiches the waist elastic members 23, 33, but also may join three or more layered sheets including sheets overlaid on the pair of sheets.

The front waist portion 20 and the back waist portion 30 have substantially the same configuration. Thus, only the front waist portion 20 will be described as a representative for both. In the present embodiment, the front waist portion 20 (stretchy sheet 40) has the plurality of joining-portion rows R in which the plurality of joining portions 50 are arranged in the vertical direction. The plurality of joining-portion rows R are arranged at intervals in the lateral direction. As shown in FIG. 4, the joining-portion rows R each extend in the vertical direction while meandering with respect to the lateral direction. That is, as shown in FIG. 6, the positions of the joining portions 50 that are arranged adjacently in the vertical direction are misaligned in the lateral direction.

On the other hand, the positions of the joining portions 50 that are arranged adjacently in the lateral direction are aligned in the vertical direction. Thus, as shown in FIG. 5A, the positions of spaces through which the waist elastic member 23 passes, between the joining portions 50 that are arranged adjacently in the lateral direction, are aligned in the vertical direction. This can arrange the waist elastic member 23 along the lateral direction. However, the positions of the joining portions 50 that are arranged adjacently in the lateral direction may be misaligned in the vertical direction, or the waist elastic member 23 may pass between the joining portions 50 that are arranged at different positions in the vertical direction. Even in these cases, the front waist portion 20 can be given stretchability in the lateral direction.

In the present embodiment, the front waist portion 20 (stretchy sheet 40) has joining-portion groups 50G in which a predetermined number of joining portions 50 are concentrated in each of the joining-portion rows R. The joining-portion groups 50G are arranged at intervals in the vertical direction. Details of the joining-portion groups 50G will be described later.

As shown in FIG. 5A, two upper and lower joining portions 50 that are located on upper and lower sides of the waist elastic member 23 in the vertical direction are also referred to as a "joining-portion pair 51" (a pair of joining portions). The upper and lower joining portions 50 that constitute the joining-portion pair 51 are arranged with a gap GH 50 in the vertical direction. The dimension of the gap GH50 is set to be equal to or slightly greater than an outer diameter d1 of the waist elastic member 23 in a state where the front waist portion 20 is stretched maximally in the lateral direction (GH50 ≧ d1).

However, the thickness of the waist elastic member 23 increases as the waist elastic member 23 contracts in the lateral direction. Then, as shown in FIG. 5B, the gap GH50 between the upper and lower joining portions 50 of the joining-portion pair 51 is set to be smaller than a maximum outer diameter d2 of the waist elastic member 23 in a state where the waist elastic member 23 contracts in the lateral direction, i.e., in a natural state of the front waist portion 20 (GH50 < d2). Accordingly, the waist elastic member 23 in a state of contracting in the lateral direction is sandwiched between the joining portions 50 of the joining-portion pair 51 (expansion in the vertical direction is restricted). This restricts the position (movement) of the waist elastic members 23 in the lateral direction and the vertical direction relative to the exterior top sheet 21 and the front-side exterior back sheet 22.

Thus, without using an adhesive for fixing the waist elastic members 23 to the exterior top sheet 21 and the front-side exterior back sheet 22, the shifting of the waist elastic members 23 can be suppressed, thereby maintaining the stretchability of the front waist portion 20. That is, with the use of the joining portions 50, the waist elastic members 23 can be attached to the exterior top sheet 21 and the front-side exterior back sheet 22 without using an adhesive or with reduced amounts of the adhesive. This can suppress the deterioration of flexibility in the front waist portion 20 (stretchy sheet 40) due to hardening of the adhesive. It is also possible to suppress the deterioration of the elastic characteristics of the waist elastic members 23 due to hardening of the adhesive.

In the front waist portion 20, the waist elastic members 23 are fixed to the exterior top sheet 21 and the front-side exterior back sheet 22 at the side joining portions SS. Specifically, when the diaper 1 is manufactured, the side joining portions SS are formed in a state where the waist elastic members 23 in the stretched state are sandwiched between the exterior top sheet 21 and the front-side exterior back sheet 22, and the side joining portions SS fix the end portions of each waist elastic member 23, respectively. As a result, even in the case where the front waist portion 20 is greatly stretched at a time of wearing of the diaper 1, the waist elastic members 23 can be prevented from coming out of the gap between the joining portions 50 of the joining-portion pair 51, thereby maintaining the stretchability of the front waist portion 20.

The relationship between the gap GH50 of the joining-portion pair 51 and the outer diameters d1, d2 of each waist elastic member 23 is not limited to the above-mentioned relationship. For example, a part of each waist elastic member 23 may be overlapped with the joining portions 50 to the extent that the waist elastic members 23 do not break. Even in this case, the waist elastic members 23 in a state of contracting in the lateral direction are sandwiched between the joining-portion pair 51 to restrict the positions of the waist elastic members 23 in the lateral direction and the vertical direction relative to the exterior top sheet 21 and the front-side exterior back sheet 22.

For restricting the position, all of the waist elastic members 23 (elastic members) arranged in the front waist portion 20 (stretchy sheet 40) need not be sandwiched between the joining-portion pair 51. The position of at least some of the waist elastic members 23 only needs to be restricted by the joining-portion pair 51, and some of the waist elastic members 23 may be fixed to the exterior top sheet 21 and the front-side exterior back sheet 22 using an adhesive.

### Joining-portion Groups 50G and Non-arrangement Regions Np

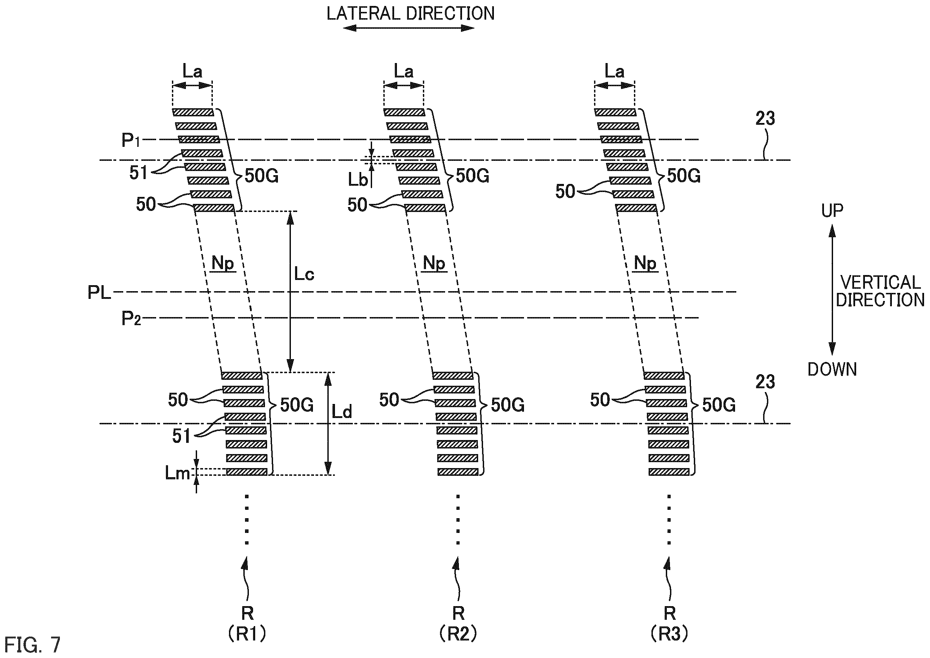

FIG. 7 is an enlarged view of parts of the joining-portion rows R of FIG. 6. As described above, the front waist portion 20 (stretchy sheet 40) has the plurality of joining-portion rows R in which the plurality of joining portions 50 are arranged in the vertical direction. In the present embodiment, the joining portions 50 constitute the joining-portion groups 50G, and the joining-portion groups 50G each include the plurality of joining portions 50. Specifically, each of the joining-portion groups 50G is an aggregate of the plurality of joining portions 50 that are arranged at intervals in the vertical direction. In such an aggregate, each of the joining-portion groups 50G is a region surrounded by: a line extending vertically and connecting left ends of the joining portions 50; a line extending vertically and connecting right ends of the joining portions 50; the upper end of the joining portion 50 arranged at the uppermost position; and the lower end of the joining portion 50 arranged at the lowermost position. As shown in FIG. 7, in a state where the front waist portion 20 is stretched in the lateral direction, in each of the joining-portion groups 50G, a vertical length Lb between the joining portions 50 that are adjacent to each other in the vertical direction is shorter than a lateral length La of each joining portion 50 (La > Lb).

In the present embodiment, each of the joining-portion groups 50G has eight joining portions 50, but the number of joining portions 50 is not limited thereto. The number of joining portions 50 included in each of the joining-portion groups 50G need not be the same. It is preferable that each of the joining-portion groups 50G includes at least four or more joining portions 50, and more preferable that each of the joining-portion groups 50G includes five or more joining portions 50.

For example, in the case where the joining-portion group 50G includes four joining portions 50, it is preferable that a vertical length Ld of each joining-portion group 50G is at least 1.0 mm or more. In such a case, it is preferable that a vertical length Lm of each joining portion 50 is 0.1 mm or more, and the vertical length Lb (i.e., the length of the above-mentioned gap GH50) between the joining portions 50 that are adjacent to each other in the vertical direction is 0.25 mm or more. For example, in the case where the joining-portion group 50G includes five joining portions 50, it is preferable that the vertical length Ld of each joining-portion group 50G is at least 2.0 mm or more. In such a case, it is preferable that a vertical length Lm of each joining portion 50 is 0.25 mm or more, and the vertical length Lb between the joining portions 50 that are adjacent to each other in the vertical direction is 0.3 mm or more. In addition, the vertical length Lm of each joining portion 50 is shorter than the lateral length La of each joining portion 50.

Each of the joining-portion groups 50G includes at least the pair of joining portions 50 (the above-described joining-portion pair 51) sandwiching the waist elastic member 23 (elastic member). Each of the joining-portion groups 50G may include two or more pairs of joining portions 50. In the joining-portion groups 50G of the present embodiment, the plurality of joining portions 50 are provided and serve as the pairs of joining portions 50 that each sandwich the waist elastic member 23. Thus, even when shifting of the waist elastic member 23 occurs during the manufacturing process, the waist elastic member 23 can be sandwiched and fixed between other joining portions 50 adjacent to each other in the vertical direction (between another pair of the joining portions 50). As a result, it is suppressed that the waist elastic member 23 is fixed while being significantly shifted from the stretch direction of the waist elastic member 23. The stretchability of the waist elastic member 23 in the stretch direction thereof can be maintained, and thus, a stable fit is likely to be exhibited when the diaper 1 is put on.

In the present embodiment, each joining-portion group 50G includes eight joining portions 50. Thus, each joining-portion group 50G has seven gaps between the joining portions 50 that are arranged adjacently in the vertical direction, and the waist elastic member 23 is sandwiched at a central gap between the pair of joining portions 50 arranged in the central portion of each joining-portion group 50G in the vertical direction. However, the position of the waist elastic member 23 is not limited thereto. The waist elastic member 23 may be arranged between another pair of joining portions 50 other than the pair of joining portions 50 at the central portion in each joining-portion group 50G.

As shown in FIG. 4, in the front waist portion 20, vertical distances between the waist elastic members 23 are each shortened at an upper end portion 20A in the vertical direction. Accordingly, in the upper end portion 20A, as compared with a lower portion 20B located below the upper end portion 20A, vertical distances between the joining-portion groups 50G that are arranged adjacently in the vertical direction are each shortened, and the number of joining-portion groups 50G (joining portions 50) is thus greater. FIGS. 6 and 7 each illustrate parts of the joining-portion rows R in the lower portion 20B described above.

In order to form aesthetic wrinkles along the product-longitudinal direction (here, along the vertical direction of the diaper 1) at a time of contraction of elastic members (such as the waist elastic members 23) arranged around the waist in an underpants-shaped absorbent article such as a disposable diaper, a method has been conventionally considered in which a plurality of joining-portion rows arranged in the vertical direction are formed at intervals in the lateral direction, as described above. On the other hand, rising of the aesthetic wrinkles extending along the vertical direction may increase the friction force against the skin. In addition, the wrinkles formed in this way are unlikely to be compressed, which may cause discomfort during usage.

In this regard, in the present embodiment, the following configuration is provided. FIG. 7 illustrates, out of the plurality of joining-portion rows R of FIG. 6, three joining-portion rows R (a first joining-portion row R1, a second joining-portion row R2, and a third joining-portion row R3) that are adjacently arranged in the lateral direction. The three joining-portion rows R are located in the lower portion 20B of the front waist portion 20 (FIG. 4). As shown in FIG. 7, the plurality of joining-portion groups 50G constitute the first joining-portion row R1, the second joining-portion row R2, and the third joining-portion row R3 that are arranged in the vertical direction. Then, a non-arrangement region Np is located between the joining-portion groups 50G that are arranged adjacently in the vertical direction, and the non-arrangement region Np is a region where the joining-portion groups 50G are not arranged. In this case, a vertical length Lc of the non-arrangement region Np is longer than the vertical length Lb between the pair of joining portions 50 (Lc > Lb). In addition, in a certain position P₁ in the vertical direction, the joining portion 50 in the first joining-portion row R1, the joining portion 50 in the second joining-portion row R2, and the joining portion 50 in the third joining-portion row R3 are overlapped with each other. In another certain position P₂ in the vertical direction, the non-arrangement region Np in the first joining-portion row R1, the non-arrangement region Np in the second joining-portion row R2, and the non-arrangement region Np in the third joining-portion row R3 are overlapped with each other.

As described above, the respective joining portions 50 in the three rows: the first joining-portion row R1; the second joining-portion row R2; and the third joining-portion row R3 that are arranged adjacently in the lateral direction are formed at a common position (P₁) in the vertical direction. Accordingly, the aesthetic winkles along the vertical direction are likely to be formed on two laterally-outward sides of each joining portion 50 when the waist elastic members 23 contract. Since the joining-portion groups 50G are not arranged in the non-arrangement regions Np, the non-arrangement regions Np are likely to be compressed. In addition, the non-arrangement regions Np in the three rows: the first joining-portion row R1; the second joining-portion row R2; and the third joining-portion row R3 that are arranged adjacently in the lateral direction are located at a common position in the vertical direction. As a result, a space along the lateral direction is likely to be formed, which can ensure softness. The above-described configuration improves flexibility of the front waist portion 20 (stretchy sheet 40) to reduce discomfort during usage.

As shown in FIG. 7, in each of the joining-portion groups 50G, the vertical length Ld is longer than the lateral length La (Ld > La). As described above, the joining-portion groups 50G are each long in the vertical direction, thereby being easier to form the wrinkles extending along the vertical direction (vertical winkles).

As shown in FIG. 7, the vertical length Lc of each non-arrangement region Np is longer than the vertical length Ld of each joining-portion group 50G (Lc > Ld). As described above, the length of each non-arrangement region Np where the joining-portion groups 50G are not arranged is long, which forms more spaces that are likely to be compressed to improve flexibility.

The vertical length Lc of each non-arrangement region Np is longer than the vertical length Ld of each joining-portion group 50G, but preferably three times or less than the vertical length Ld. In the case where the non-arrangement regions Np without the joining-portion groups 50G are each excessively long, the unjoined spaces each become long. As a result, the aesthetic winkles along the vertical direction are less likely to be formed at a time of contraction. Therefore, the vertical length Lc of each non-arrangement region Np is set to be three times or less than the vertical length Ld of each joining-portion group 50G to prevent the vertical distance between the joining-portion groups 50G from being excessively long. This can promote formation of the wrinkles extending along the vertical direction using the joining-portion group 50G.

As described above, each of the waist elastic members 23 is sandwiched between the pair of joining portions 50, but the vertical distance between the waist elastic members 23, 23 that are arranged adjacently in the vertical direction is preferably 4 mm or more. In the case where the vertical distance between the waist elastic members 23, 23 that are arranged adjacently in the vertical direction is excessively short, the stiffness between the waist elastic members 23, 23 may become excessively high to cause discomfort at a time of wearing of the diaper 1. On the other hand, in the case where the vertical distance between the waist elastic members 23, 23 that are arranged adjacently in the vertical direction is set to be 4 mm or more, the diaper 1 is easier to be pulled up by the wearer's fingers being inserted between the waist elastic members 23, 23 at a time of wearing of the diaper 1. In addition, discomfort during usage can be reduced without excessively increasing the stiffness.

When a central position PL (FIG. 7) refers to a vertically-central portion between the waist elastic members 23, 23 that are arranged adjacently in the vertical direction, it is preferable that each non-arrangement region Np where the joining-portion groups 50G are not arranged spans the central position PL. More preferably, each non-arrangement region Np is located in a position that is shifted above the central position PL. In each non-arrangement region Np where the joining-portion groups 50G are not arranged, the difference in stiffness does not occur and a tear is less likely to be torn. Such a non-arrangement region Np is located in a position spanning the central position PL or in a position shifted above the central position PL. As a result, the waist portions (the front waist portion 20 and the back waist portion 30) are less likely to be torn when the wearer inserts his or her fingers between the waist elastic members 23, 23 to pull up the diaper 1.

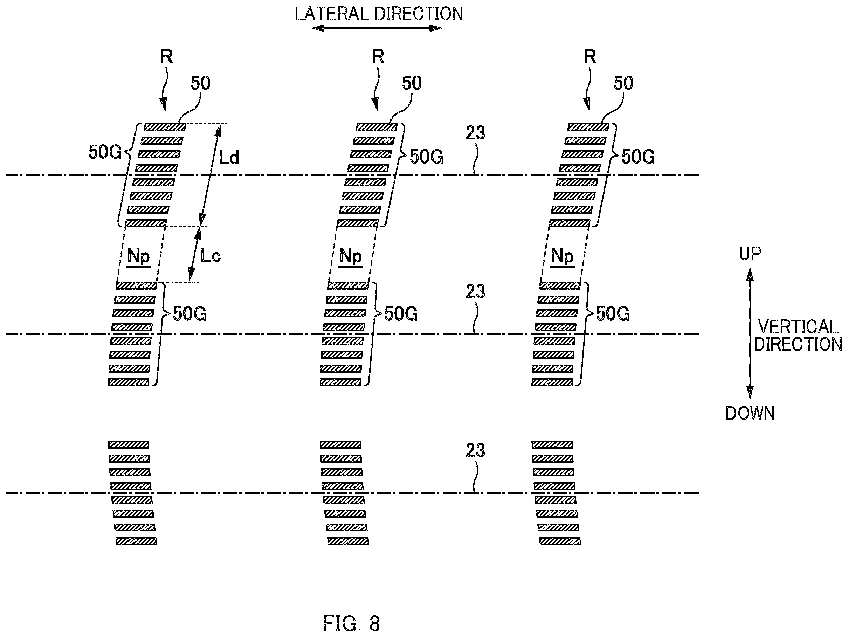

FIG. 8 is a diagram illustrating the joining-portion rows R in the upper end portion 20A shown in FIG. 4. As shown in FIG. 8, in the upper end portion 20A of the front waist portion 20, the vertical length Lc of each non-arrangement region Np is shorter than the vertical length Ld of each joining-portion group 50G (Lc < Ld). In the front waist portion 20, the upper end portion 20A is a portion where curling or damage is likely to occur during usage. In such an upper end portion 20A, the vertical length of each joining-portion group 50G is longer than that of each non-arrangement region Np, thereby being likely to increase the stiffness and to form the aesthetic winkles along the vertical direction. This improves the fit while being less likely to be damaged.

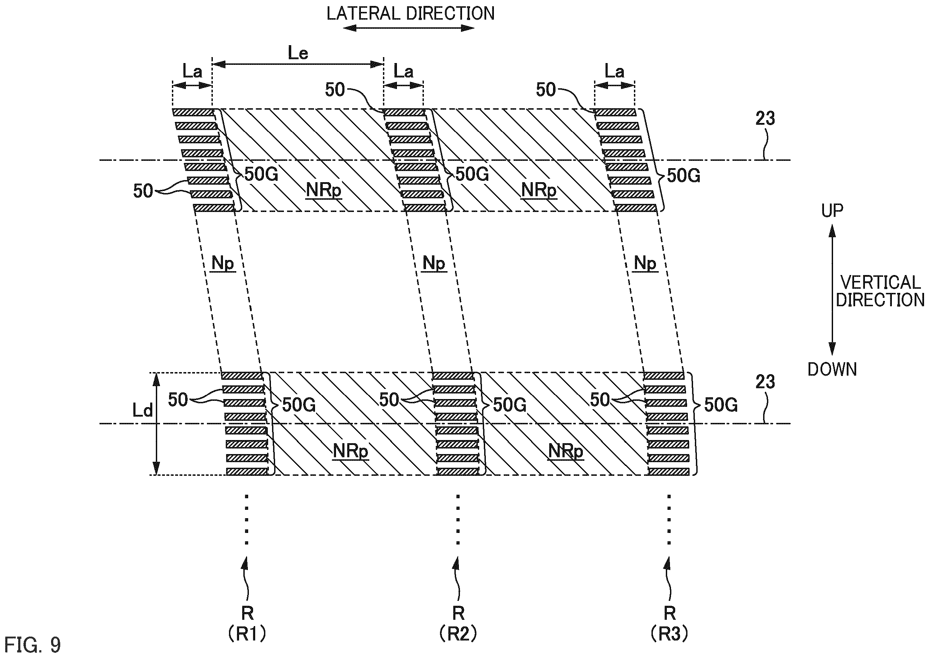

FIG. 9 is a diagram illustrating regions between the joining-portion groups 50G arranged adjacently in the lateral direction. As illustrated in FIG. 9, an inter-row non-arrangement region NRp is located between the joining-portion groups 50G that are arranged adjacently in the lateral direction (e.g., in FIG. 9, between laterally-adjacent joining-portion groups 50G in the first joining-portion row R1 and in the second joining-portion row R2, and between laterally-adjacent joining-portion groups 50G in the second joining-portion row R2 and the third joining-portion row R3), and the inter-row non-arrangement region NRp is a region where the joining-portion groups 50G are not arranged. In this case, a lateral length Le of the inter-row non-arrangement region NRp is longer than the vertical length Ld of each joining-portion group 50G. In the case where the lateral length Le of the inter-row non-arrangement region NRp is short, the wrinkles extending along the vertical direction are likely to be formed finely upon contraction of the waist elastic members 23. This may result in hard texture. However, in the present embodiment, the lateral length Le of the inter-row non-arrangement region NRp is long, thereby making large wrinkles extending along the vertical direction likely to be formed upon contraction of the waist elastic members 23. This can improve the texture.

It is preferable that the lateral length Le of the inter-row non-arrangement region NRp is longer than the vertical length Ld of each joining-portion group 50G, but is three times or less than the vertical length Ld. In the case where the lateral length of the inter-row non-arrangement region NRp where the joining-portion groups 50G are not provided is excessively long, the unjoined space becomes long. As a result, aesthetic winkles are less likely to be formed along the vertical direction at a time of contraction. Thus, the lateral length Le of the inter-row non-arrangement region NRp is three times or less than the vertical length Ld of each joining-portion group 50G, so that the distance between the joining-portion groups 50G arranged adjacently in the lateral direction can be prevented from being excessively large. This can promote the formation of the wrinkles extending along the vertical direction using the joining-portion groups 50G.

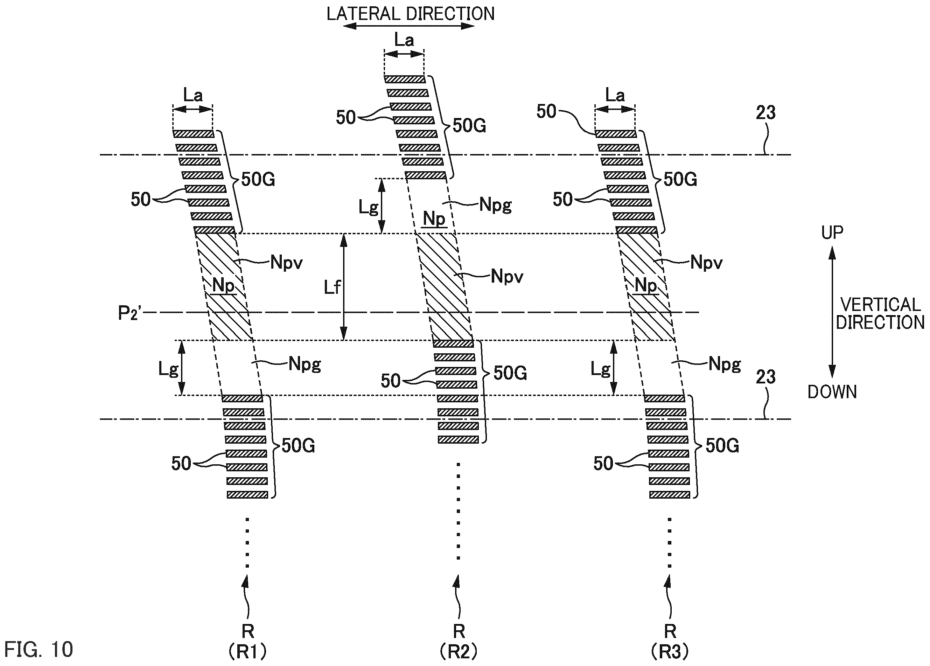

FIG. 10 is a diagram illustrating an arrangement of joining-portion rows R according to a modified example. In the embodiment shown in FIGS. 7 and 8, the respective upper and lower ends of: the non-arrangement region Np in the first joining-portion row R1; the non-arrangement region Np in the second joining-portion row R2; and the non-arrangement region Np in the third joining-portion row R3 are located at the same position with respect to the vertical direction. That is, the non-arrangement regions Np are overlapped with each other with respect to the vertical direction. However, the positions of the joining-portion groups 50G and the non-arrangement regions Np are not limited thereto.

For example, in the modified example shown in FIG. 10, the vertical position of the joining-portion group 50G and the vertical position of the non-arrangement region Np in the second joining-portion row R2 are shifted from the vertical positions of the joining-portion groups 50G and the vertical positions of the non-arrangement regions Np in the first joining-portion row R1 and the third joining-portion row R3. Here, out of the non-arrangement regions Np in the first joining-portion row R1, in the second joining-portion row R2, and in the third joining-portion row R3 that are overlapped with each other in a position P₂' in the vertical direction shown in FIG. 10, the non-arrangement regions Np in the first joining-portion row R1 and the second joining-portion row R2 (two non-arrangement regions) include: overlap regions Npv where the non-arrangement regions Np in the first joining-portion row R1 and the second joining-portion row R2 are overlapped with each other with respect to the vertical direction; and non-overlap regions Npg where the non-arrangement regions Np in the first joining-portion row R1 and the second joining-portion row R2 are not are overlapped with each other with respect to the vertical direction. In this case, it is preferable that a vertical length Lf of the overlap region Npv is longer than a vertical length Lg of the non-overlap region Npg (Lf > Lg). As in the above case, in the non-arrangement regions Np in the second joining-portion row R2 and the third joining-portion row R3 (two non-arrangement regions), it is preferable that the vertical length Lf of each of the overlap regions Npv is longer than the vertical length Lg of each of the non-overlap regions Npg. The unfixed non-arrangement regions Np are likely to be compressed, and do not have the joining portions 50. Thus, such non-arrangement regions Np are each a region where a space having good texture is likely to be formed. The vertical length Lf of each overlap region Npv where the non-arrangement regions Np are overlapped with each other is longer than the vertical length Lg of each non-overlap region Npg, and the overlap regions Npv are arranged in the lateral direction, thereby making a larger space likely to be secured. Such a configuration can improve flexibility.

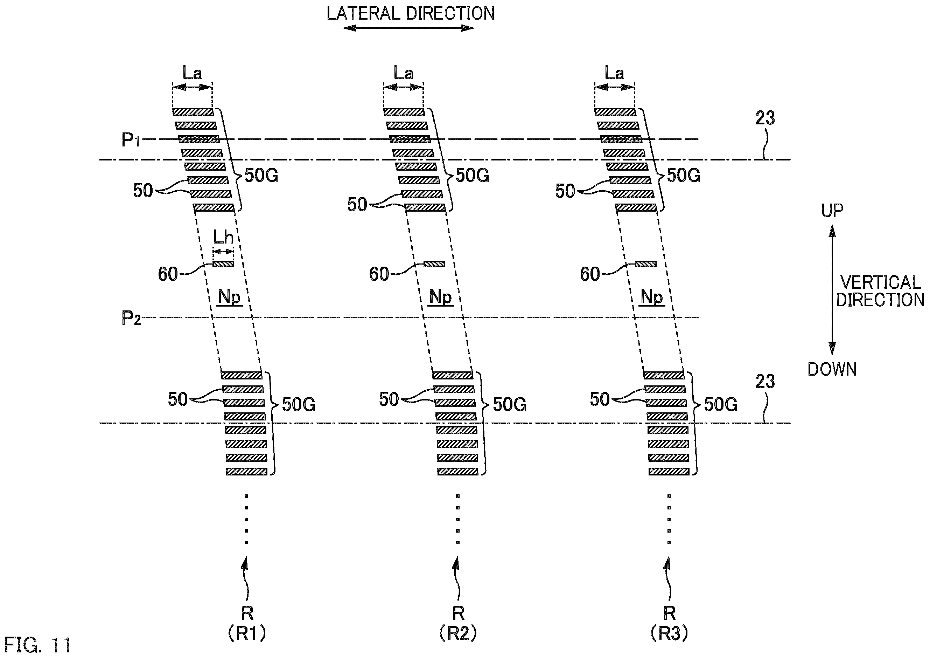

FIG. 11 is a diagram illustrating joining-portion rows R according to a modified example. The modified example in FIG. 11 describes an example of parts of joining-portion rows R in the front waist portion 20, as with the above-mentioned embodiment. In the present modified example, each of the joining-portion rows R includes an auxiliary joining portion 60 that is provided in a part of each non-arrangement region Np. The auxiliary joining portion 60 is different from the joining portions 50 and joins the exterior top sheet 21 and the front-side exterior back sheet 22 (a pair of sheets). Examples of a joining method using the auxiliary joining portion 60 include heat welding, ultrasonic welding, and joining with an adhesive. In the case of a long distance between the joining-portion groups 50G that are arranged adjacently in the vertical direction (i.e., the vertical length of each non-arrangement region Np), the regular wrinkles extending along the vertical direction may be unlikely to be formed by the pair of sheets at a time of contraction of the waist elastic members 23. In such a case, as in the present modified example, the auxiliary joining portion 60 is provided in a part of each non-arrangement region Np, which makes regular wrinkles extending along the vertical direction likely to be formed. The auxiliary joining portion 60 only needs to serve as a portion that assists formation of such regular wrinkles, and thus, a lateral length Lh of the auxiliary joining portion 60 may be shorter than the lateral length La of each joining-portion group 50G, as shown in the modified example. The length Lh of the auxiliary joining portion 60 is shortened to avoid deterioration of the texture, which easily maintains flexibility. The present disclosure is not limited thereto, the lateral length Lh of the auxiliary joining portion 60 may be longer than the lateral length La of each joining-portion group 50G. In the present modified example, the auxiliary joining portion 60 is formed in a rectangular shape, but the shape thereof is not limited to the rectangular shape, and any shape can be adopted.

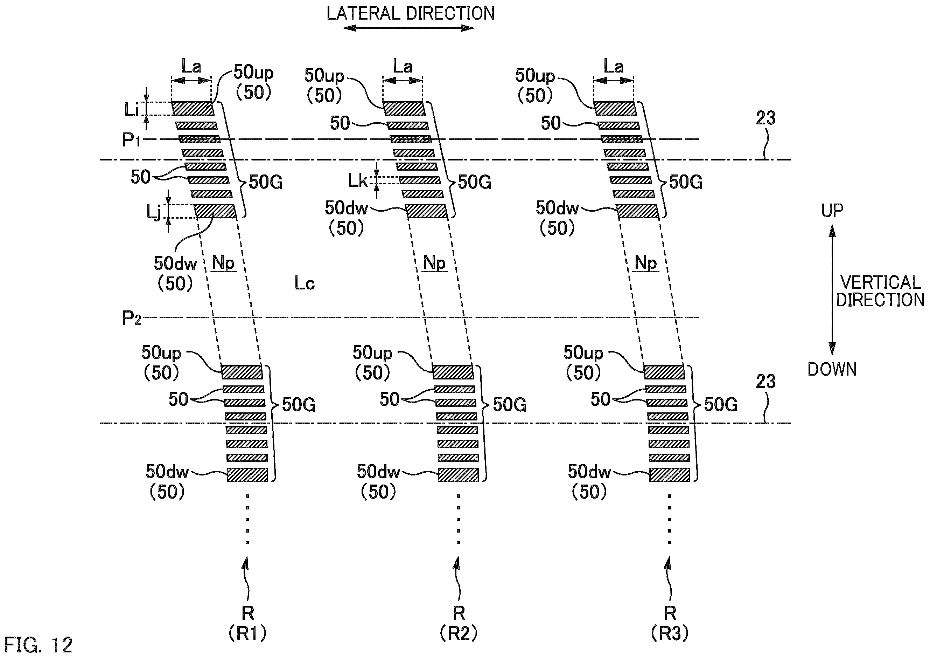

FIG. 12 is a diagram illustrating joining-portion rows R according to another modified example. The modified example in FIG. 12 describes parts of the joining-portion rows R in the front waist portion 20, as with the above-mentioned embodiment. In the above-mentioned embodiment, each of the joining-portion groups 50G has the plurality of joining portions 50 each having the same dimension, but the present disclosure is not limited thereto. In the modified example shown in FIG. 12, in each joining-portion group 50G, the dimensions of the joining portions 50 that are located on two vertical ends thereof are larger than those of the joining portions 50 other than the joining portions 50 on the two vertical ends thereof. Specifically, out of the joining portions 50 on the two vertical ends thereof in each joining-portion group 50G, the joining portion 50 on an upper-vertical end thereof is referred to as an upper-end joining portion 50up, and the joining portion 50 on a lower-vertical end thereof is referred to as a lower-end joining portion 50dw. In such a case, a vertical length Li of the upper-end joining portion 50up and a vertical length Lj of the lower-end joining portion 50dw are longer than a vertical length Lk of each joining portion 50 other than the upper-end joining portion 50up and the lower-end joining portion 50dw in the common joining-portion group 50G.

In each joining-portion group 50G, the waist elastic member 23 is arranged between any one pair of joining portions 50 in each joining-portion group 50G. However, in such a case, in the manufacturing process, when the waist elastic member 23 is attempted to be arranged on one of the two vertical ends of the joining portions 50, i.e., between the pair of joining portions 50 including the uppermost joining portion 50 or the lowermost joining portion 50, the waist elastic member 23 may be arranged at a shifted position. In this regard, in each joining-portion group 50G, the vertical lengths Li, Lj of the joining portions 50 on two vertical ends (the upper-end joining portion 50up and the lower-end joining portion 50dw) increase, and thus, the waist elastic member 23 that is arranged at an excessively shifted position can be cut by the upper-end joining portion 50up or the lower-end joining portion 50dw. This can avoid the arrangement of the waist elastic member 23 in an unexpected position.

As described above, FIGS. 6 to 9 each illustrate a state where the front waist portion 20 (stretchy sheet 40) is stretched in the lateral direction. Each of the non-arrangement regions Np is a region where the joining portions 50 are not arranged, that is, the unfixed region. Thus, such non-arrangement regions Np are compressed, thereby making spaces be easily formed. More specifically, at a time of contraction of the waist elastic members 23 (33) (elastic members), the wrinkles extending along the vertical direction are formed in the diaper 1 on two lateral sides of each joining-portion group 50G, but the wrinkles also extend along the vertical direction on two lateral sides of each non-arrangement region Np. Similarly, at a time of contraction of the waist elastic members 23, protrusions (unillustrated) are formed in each of the non-arrangement regions Np (e.g., the non-arrangement region Np in the first joining-portion row R1, the non-arrangement region Np in the second joining-portion row R2, and the non-arrangement region Np in the third joining-portion row R3). Such protrusions are formed by the exterior top sheet 21 and the front-side exterior back sheet 22 (pair of sheets) protruding toward one side or the other side of the front waist portion 20 (stretchy sheet 40) in the thickness direction thereof. In each protrusion, two sheets (the exterior top sheet 21 and the front-side exterior back sheet 22) may protrude in the same direction (protrude toward one side or the other side), or the two sheets may protrude in different directions (one of the sheets may protrude toward one side, the other of the sheets may protrude toward the other side). The joining portions 50 are not present in the protrusion, and thus, the protrusion has comfortable texture. Such a plurality of protrusions having comfortable texture are formed to improve flexibility of the front waist portion 20 (stretchy sheet 40).

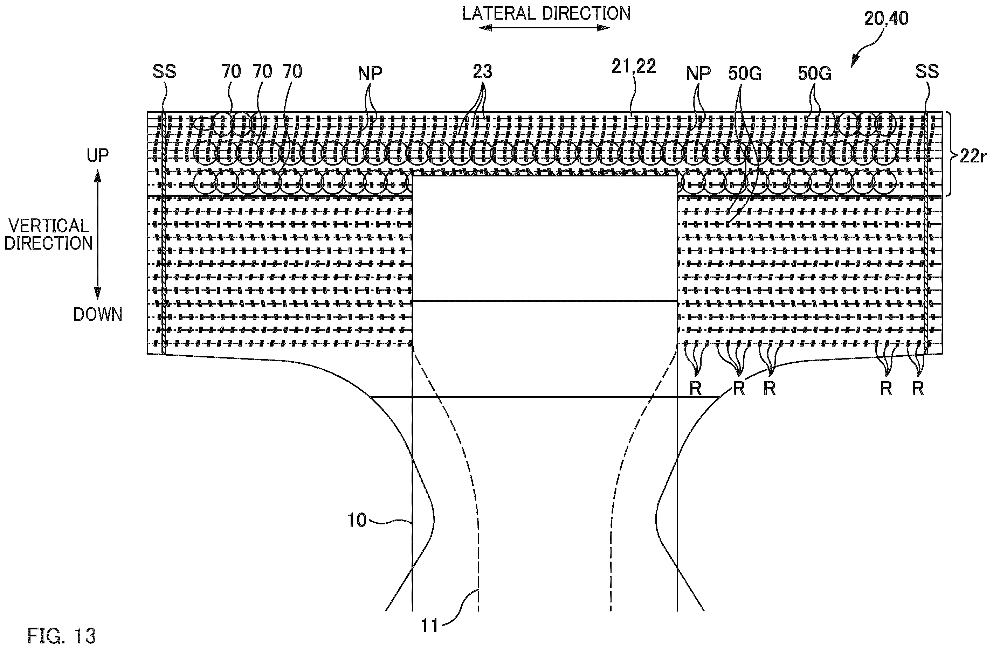

FIG. 13 is a diagram illustrating adhesion portions 70 in a folded portion 22r. The diaper 1 has folded portions 22r and 32r. The folded portions 22r and 32r are formed by at least one of the pair of sheets including the exterior top sheet 21 and the front-side exterior back sheet 22 (back-side exterior back sheet 32) (in the above-mentioned embodiment, the front-side exterior back sheet 22 and the back-side exterior back sheet 32) being folded toward the inner surfaces (skin-side surfaces) of the waist portions 20 and 30 (see FIGS. 2 and 3). The folded portions 22r and 32r respectively adhere to the inner surfaces (skin-side surfaces) of the waist portion 20 and 30 via the adhesion portions 70 to which an adhesive such as a hot-melt adhesive is applied. Examples of the application pattern of the adhesive include a circular pattern shown in FIG. 13, but the present disclosure is not limited thereto. Other application patterns may be used.

As shown in FIG. 13, in the case where portions other than the adhesion portions 70 in the folded portion 22r are referred to as non-adhesion portions, the diaper 1 has a portion where such non-adhesion portions are overlapped with the non-arrangement regions Np in a plan view. Such portions where the non-adhesion portions in the folded portion 22r are overlapped with the non-arrangement regions Np are regions where the adhesion portions 70 and the joining portions 50 for adhesion of materials are not present while increasing the number of stacked materials in the thickness direction, and such portions are also more likely to be compressed. Such compressible regions can improve the texture of the waist portion 20.

In the diaper 1, at least one of the pair of waist portions 20 and 30 has another sheet (unillustrated) other than the pair of sheets (the exterior top sheet 21 and the front-side exterior back sheet 22 (back-side exterior back sheet 32)) such that the other sheet is overlapped with the above-mentioned pair of sheets in the thickness direction (in the front-back direction in the case of the underpants-shaped diaper 1 in FIG. 1). It is preferable that the other sheet adheres to the skin-side surfaces of the pair of sheets via adhesion portions (unillustrated) to which an adhesive such as a hot-melt adhesive is applied, e.g., the adhesion portions 70. The above-mentioned portion where the pair of sheets and the other sheet are arranged so as to be overlapped with each other in the thickness direction is referred to as a stacked portion. In this case, there is a portion where the non-adhesion portions other than the adhesion portions in the stacked portion are overlapped with the non-arrangement regions Np in a plan view, thereby making the texture of the waist portion 20 (30) likely to be improved, as with the above-mentioned folded portion 22r. That is, the portion where the non-adhesion portions in the stacked portion are overlapped with the non-arrangement regions Np is a region where the joining portions (adhesion portions) for joining (adhesion) are not present while increasing the number of stacked materials. Such a region is thus likely to be compressed. Such a compressible region can improve the texture.

Returning to FIG. 13, the folded portion 22r also has a portion where the adhesion portions 70 for adhesion of the folded portion 22r and the non-arrangement regions Np are overlapped with each other in a plan view. Since the adhesive is applied to the adhesion portions 70, the adhesion portions 70 are each a region having high stiffness. However, the region where the adhesion portions 70 and the non-arrangement regions Np are overlapped with each other is more likely to be compressed, as compared with the portion where the adhesion portions 70 and the joining-portion groups 50G are overlapped with each other in a plan view. Thus, the region where the adhesion portions 70 and the non-arrangement regions Np are overlapped with each other prevents the adhesion portions 70 from having excessively high stiffness, which can avoid deterioration in the texture.

As shown in FIG. 13, the adhesion portions 70 in the folded portion 22r are provided in a position away from the side joining portions SS in the lateral direction. Specifically, it is preferable that the adhesion portions 70 in the folded portion 22r are spaced from the side joining portions SS inwardly in the lateral direction, at a distance of about 10 mm to 20 mm. The side joining portions SS are each a region having high stiffness, but the adhesion portions 70 are not provided in the vicinity of the side joining portions SS, which makes large and flexible wrinkles likely to be formed along the joining-portion rows R in the vicinity of the side joining portions SS. This can avoid deterioration in the texture even in the vicinity of the side joining portions SS having high stiffness.

Furthermore, in the present embodiment, the diaper 1 has a portion where the absorbent main body 10 and the non-arrangement regions Np are overlapped with each other in a plan view (such an overlap portion is not shown in FIG. 4, but the joining-portion groups 50G and the non-arrangement regions Np are continuously provided on the non-skin side of the absorbent main body 10 in FIG. 4). The absorbent main body 10 has higher stiffness, but the portion where the absorbent main body 10 and the non-arrangement regions Np are overlapped with each other in a plan view is a portion where the waist portions 20, 30 are likely to be compressed. This can reduce discomfort when the wearer puts on the diaper 1.

As described above, the front waist portion 20 and the back waist portion 30 of the present embodiment include the exterior top sheet 21 and the front-side exterior back sheet 22 that are joined by the plurality of joining portions 50, or the exterior top sheet 21 and the back-side exterior back sheet 32 that are joined by the plurality of joining portions 50. It is preferable that the total basis weight of the exterior top sheet 21 and the front-side exterior back sheet 22 (or the exterior top sheet 21 and the back-side exterior back sheet 32) constituting the waist portions (front and back waist portions 20, 30) is 30 g/m² or less. The total basis weight of the waist portions (front and back waist portions 20, 30) that constitute the sheet members (the exterior top sheet 21 and the front-side exterior back sheet 22, or the exterior top sheet 21 and the back-side exterior back sheet 32) is set to be 30 g/m² or less, which can easily pull up the diaper 1 at a time of wearing thereof. In addition, in the sheet members, the higher the total basis weight is, the higher the stiffness is. When the plurality of joining portions 50 are formed on the sheet members having high stiffness using a welding method, the stiffness may be excessively high. However, the total basis weight is equal to or less than the above-mentioned value, which can suppress that the stiffness is excessively high to reduce discomfort during usage.

As shown in FIG. 2, the diaper 1 of the present embodiment has an integrated shape in which the exterior body 18 continues from the front waist portion 20 to the back waist portion 30. Thus, when the front waist portion 20 or the back waist portion 30 is held to pull up the diaper 1, a pulling force is transmitted toward the crotch portion 35. As a result, the crotch portion 35 is easily pulled up. Therefore, the fit of the product is further improved.

### Others

Although the above embodiment is simply to facilitate understanding of the present disclosure and is not in any way to be construed as limiting the present disclosure. The present disclosure may be variously changed or altered without departing from its gist and encompass equivalents thereof.

### [Reference Signs List]

1 underpants-shaped disposal diaper
10 absorbent main body
11 absorbent core
12 top sheet
13 back sheet
14 exterior sheet
15 leg elastic member
16 leak-proof-wall elastic member
18 exterior body
20 waist portion (front waist portion)
20A upper end portion
20B lower portion
21 exterior top sheet
22 front-side exterior back sheet
22r folded portion
23 waist elastic member
30 waist portion (back waist portion)
32 back-side exterior back sheet
32r folded portion
33 waist elastic member
34 back leg elastic member
35 crotch portion
40 stretchy sheet
50 joining portion
50dw lower-end joining portion
50G joining-portion group
50up upper-end joining portion
51 joining-portion pair
60 auxiliary joining portion
70 adhesion portion

## Claims

1. A stretchy sheet (40) having a vertical direction and a lateral direction that intersect each other,
the stretchy sheet (40) comprising:
a plurality of elastic members that are located between a pair or sheets joined by a plurality of joining portions (50),
the plurality of elastic members (23) being arranged at intervals in the vertical direction,
the plurality of elastic members being capable of stretching and contracting in the lateral direction; and
a joining-portion group (50G) that includes at least one pair of the joining portions (50) sandwiching each of the elastic members,
a plurality of the joining-portion groups (50G) constituting a first joining-portion row, a second joining-portion row, and a third joining-portion row that are arranged in the vertical direction,
in a state where the stretchy sheet (40) is stretched in the lateral direction,
in each of the joining-portion groups (50G), a vertical length between the joining portions (50) that are arranged adjacently in the vertical direction being shorter than a lateral length of each of the joining portions (50),
a non-arrangement region (Np) being located between the joining-portion groups (50G) that are arranged adjacently in the vertical direction,
the non-arrangement region (Np) being a region where the joining-portion groups (50G) are not arranged,
a vertical length of the non-arrangement region (Np) being longer than the vertical length between the pair of the joining portions (50),
the first joining-portion row, the second joining-portion row, and the third joining-portion row being arranged adjacently in the lateral direction,
in a certain position in the vertical direction, one of the joining portions (50) in the first joining-portion row, one of the joining portions (50) in the second joining-portion row, and one of the joining portions (50) in the third joining-portion row being overlapped with each other,
in another certain position in the vertical direction, the non-arrangement region (Np) in the first joining-portion row, the non-arrangement region (Np) in the second joining-portion row, and the non-arrangement region (Np) in the third joining-portion row being overlapped with each other.

2. The stretchy sheet (40) according to claim 1, wherein
in a state where the stretchy sheet is stretched in the lateral direction,
a vertical length of each of the joining-portion groups (50G) is longer than the lateral length of each of the joining-portion groups (50G).

3. The stretchy sheet (40) according to claim 1 or 2, wherein
in a state where the stretchy sheet (40) is stretched in the lateral direction,
out of the non-arrangement region (Np) in the first joining-portion row, the non-arrangement region (Np) in the second joining-portion row, and the non-arrangement region (Np) in the third joining-portion row that are overlapped with each other in the other certain position,
two non-arrangement regions (Np) include:
an overlap region where the two non-arrangement regions are overlapped with each other with respect to the vertical direction; and
a non-overlap region where the two non-arrangement regions are not overlapped with each other with respect to the vertical direction, and
a vertical length of the overlap region is longer than a vertical length of the non-overlap region.

4. The stretchy sheet (40) according to claim 1 or 2, wherein
in a state where the stretchy sheet (40) is stretched in the lateral direction,
the vertical length of the non-arrangement region (Np) is longer than the vertical length of each of the joining-portion groups.

5. The stretchy sheet (40) according to claim 4, wherein
the vertical length of the non-arrangement region (Np) is three times or less than the vertical length of each of the joining-portion groups (50G).

6. The stretchy sheet (40) according to claim 1 or 2, wherein
in a state where the stretchy sheet (40) is stretched in the lateral direction,
the vertical length of the non-arrangement region (Np) is shorter than the vertical length of each of the joining-portion groups (50G).

7. The stretchy sheet (40) according to claim 1 or 2, wherein
in a state where the stretchy sheet (40) is stretched in the lateral direction,
an inter-row non-arrangement region is located between the joining-portion groups (50G) that are arranged adjacently in the lateral direction,
the inter-row non-arrangement region being a region where the joining-portion groups (50G) are not arranged, and
a lateral length of the inter-row non-arrangement region is longer than the vertical length of each of the joining-portion groups (50G).

8. The stretchy sheet (40) according to claim 7, wherein
the lateral length of the inter-row non-arrangement region is three times or less than the vertical length of each of the joining-portion groups (50G).

9. The stretchy sheet (40) according to claim 1 or 2, wherein
an auxiliary joining portion (50) that joins the pair of sheets together is provided in a part of the non-arrangement region,
the auxiliary joining portion (50) being different from the joining portions (50G).

10. The stretchy sheet (40) according to claim 1 or 2, wherein
in a state where the elastic members (23) contract,
the non-arrangement region (Np) in the first joining-portion row, the non-arrangement region in the second joining-portion row, and the non-arrangement region (Np) in the third joining-portion row that are overlapped with each other in the other position in the vertical direction each have a protrusion,
the protrusion protruding toward one side or another side of the stretchy sheet (40) in a thickness direction of the stretchy sheet.

11. The stretchy sheet (40) according to claim 1 or 2, wherein
each of the joining groups (50G) includes:
an upper-end joining portion (50up) referring to the joining portion (50) on an upper vertical end of each of the joining-portion groups (50G);
a lower-end joining portion (50dw) referring to the joining portion (50) on a lower vertical end of each of the joining-portion groups (50G), and
a vertical length of the upper-end joining portion (50up) and a vertical length of the lower-end joining portion (50dw) are each longer than a vertical length of each of the joining portions (50) other than the upper-end joining portion (50up) and the lower-end joining portion (50dw) in each of the joining-portion groups.

12. An underpants-shaped absorbent article (1) having a vertical direction, a lateral direction, and a front-back direction that intersect each other,
the underpants-shaped absorbent article (1) comprising:
the stretchy sheet (40) according to claim 1 or 2;
an absorbent main body (10); and
a pair of waist portions (20, 30),
the stretchy sheet (40)_being provided in at least one of the pair of waist portions (20, 30),
the vertical direction of the stretchy sheet (40) extending along the vertical direction of the underpants-shaped absorbent article (1),
the lateral direction of the stretchy sheet (40) extending along the lateral direction of the underpants-shaped absorbent article (1),
a thickness direction of the stretchy sheet (40) extending along the front-back direction of the underpants-shaped absorbent article (1),
the pair of waist portions (20, 30) being joined together by a pair of side joining portions (SS), on two lateral sides of the pair of waist portions (20, 30),
at least one of the pair of waist portions (20, 30) including a stacked portion or a folded portion (22r, 32r),
the stacked portion being a portion where the pair of sheets adheres to another sheet other than the pair of sheets via an adhesion portion (70) such that the pair of sheets and the other sheet are overlapped with each other with respect to the front-back direction,
the folded portion (22r, 32r) being a portion where at least one of the pair of sheets is folded toward an inner surface of the at least one of the pair of waist portions (20, 30) and adheres to the inner surface of the at least one of the waist portions (20, 30) via the adhesion portion (70),
the underpants-shaped absorbent article (1) including a portion where the non-arrangement region is overlapped with a non-adhesion portion other than the adhesion portion (70) in the stacked portion or a non-adhesion portion other than the adhesion portion (70) in the folded portion (22r, 32r), in a plan view.

## Patentansprüche

1. Dehnbare Lage (40), die eine vertikale Richtung und eine laterale Richtung aufweist, die einander schneiden,
wobei die dehnbare Lage (40) Folgendes umfasst:
eine Vielzahl von elastischen Elementen, die sich zwischen einem Paar von Lagen befinden, die durch eine Vielzahl von Verbindungsteilen (50) verbunden sind,
wobei die Vielzahl von elastischen Elementen (23) in Abständen in der vertikalen Richtung angeordnet ist,
sich die Vielzahl von elastischen Elementen in der lateralen Richtung dehnen und zusammenziehen kann; und
eine Verbindungsteilgruppe (50G), die mindestens ein Paar von Verbindungsteilen (50) einschließt, die jedes der elastischen Elemente einfügen,
wobei eine Vielzahl von Verbindungsteilgruppen (50G) eine erste Verbindungsteilreihe, eine zweite Verbindungsteilreihe und eine dritte Verbindungsteilreihe darstellt, die in der vertikalen Richtung angeordnet sind,
wobei in einem Zustand, in dem die dehnbare Lage (40) in der lateralen Richtung gedehnt ist,
in jeder der Verbindungsteilgruppen (50G) eine vertikale Länge zwischen den Verbindungsteilen (50), die benachbart in der vertikalen Richtung angeordnet sind, kürzer ist als eine laterale Länge von jedem der Verbindungsteile (50),
sich ein Nicht-Anordnungsbereich (Np) zwischen den Verbindungsteilgruppen (50G) befindet, die benachbart in der vertikalen Richtung angeordnet sind,
wobei der Nicht-Anordnungsbereich (Np) ein Bereich ist, in dem die Verbindungsteilgruppen (50G) nicht angeordnet sind,
eine vertikale Länge des Nicht-Anordnungsbereichs (Np) länger ist als die vertikale Länge zwischen dem Paar von Verbindungsteilen (50),
die erste Verbindungsteilreihe, die zweite Verbindungsteilreihe und die dritte Verbindungsteilreihe benachbart in der lateralen Richtung angeordnet sind,
in einer bestimmten Position in der vertikalen Richtung einer der Verbindungsteile (50) in der ersten Verbindungsteilreihe, einer der Verbindungsteile (50) in der zweiten Verbindungsteilreihe und einer der Verbindungsteile (50) in der dritten Verbindungsteilreihe miteinander überlappen,
in einer anderen bestimmten Position in der vertikalen Richtung der Nicht-Anordnungsbereich (Np) in der ersten Verbindungsteilreihe, der Nicht-Anordnungsbereich (Np) in der zweiten Verbindungsteilreihe und der Nicht-Anordnungsbereich (Np) in der dritten Verbindungsteilreihe miteinander überlappen.

2. Dehnbare Lage (40) nach Anspruch 1, wobei
in einem Zustand, in dem die dehnbare Lage in der lateralen Richtung gedehnt ist,
eine vertikale Länge von jeder der Verbindungsteilgruppen (50G) länger ist als die laterale Länge von jeder der Verbindungsteilgruppen (50G).

3. Dehnbare Lage (40) nach Anspruch 1 oder 2, wobei
in einem Zustand, in dem die dehnbare Lage (40) in der lateralen Richtung gedehnt ist,
von dem Nicht-Anordnungsbereich (Np) in der ersten Verbindungsteilreihe, dem Nicht-Anordnungsbereich (Np) in der zweiten Verbindungsteilreihe und dem Nicht-Anordnungsbereich (Np) in der dritten Verbindungsteilreihe, die in der anderen bestimmten Position miteinander überlappen,
zwei Nicht-Anordnungsbereiche (Np) Folgendes einschließen:
einen Überlappungsbereich, in dem die zwei Nicht-Anordnungsbereiche in Bezug auf die vertikale Richtung miteinander überlappen; und
einen Nicht-Überlappungsbereich, in dem die zwei Nicht-Anordnungsbereiche in Bezug auf die vertikale Richtung nicht miteinander überlappen, und
eine vertikale Länge des Überlappungsbereichs länger ist als eine vertikale Länge des Nicht-Überlappungsbereichs.

4. Dehnbare Lage (40) nach Anspruch 1 oder 2, wobei
in einem Zustand, in dem die dehnbare Lage (40) in der lateralen Richtung gedehnt ist,
die vertikale Länge des Nicht-Anordnungsbereichs (Np) länger ist als die vertikale Länge von jeder der Verbindungsteilgruppen.

5. Dehnbare Lage (40) nach Anspruch 4, wobei
die vertikale Länge des Nicht-Anordnungsbereichs (Np) das Dreifache oder weniger als die vertikale Länge von jeder der Verbindungsteilgruppen (50G) beträgt.

6. Dehnbare Lage (40) nach Anspruch 1 oder 2, wobei
in einem Zustand, in dem die dehnbare Lage (40) in der lateralen Richtung gedehnt ist,
die vertikale Länge des Nicht-Anordnungsbereichs (Np) kürzer ist als die vertikale Länge von jeder der Verbindungsteilgruppen (50G).

7. Dehnbare Lage (40) nach Anspruch 1 oder 2, wobei
in einem Zustand, in dem die dehnbare Lage (40) in der lateralen Richtung gedehnt ist,
sich ein Nicht-Anordnungszwischenreihenbereich zwischen den Verbindungsteilgruppen (50G) befindet, die benachbart in der lateralen Richtung angeordnet sind,
wobei der Nicht-Anordnungszwischenreihenbereich ein Bereich ist, in dem die Verbindungsteilgruppen (50G) nicht angeordnet sind, und
eine laterale Länge des Nicht-Anordnungszwischenreihenbereichs länger ist als die vertikale Länge von jeder der Verbindungsteilgruppen (50G).

8. Dehnbare Lage (40) nach Anspruch 7, wobei
die laterale Länge des Nicht-Anordnungszwischenreihenbereichs das Dreifache oder weniger als die vertikale Länge von jeder der Verbindungsteilgruppen (50G) beträgt.

9. Dehnbare Lage (40) nach Anspruch 1 oder 2, wobei
ein Hilfsverbindungsteil (50), das das Paar von Lagen miteinander verbindet, in einem Teil des Nicht-Anordnungsbereichs bereitgestellt ist,
wobei sich der Hilfsverbindungsteil (50) von den Verbindungsteilen (50G) unterscheidet.

10. Dehnbare Lage (40) nach Anspruch 1 oder 2, wobei
in einem Zustand, in dem die elastischen Elemente (23) zusammengezogen sind,
der Nicht-Anordnungsbereich (Np) in der ersten Verbindungsteilreihe, der Nicht-Anordnungsbereich (Np) in der zweiten Verbindungsteilreihe und der Nicht-Anordnungsbereich (Np) in der dritten Verbindungsteilreihe, die in der anderen Position in der vertikalen Richtung miteinander überlappen, jeweils eine Ausstülpung aufweisen,
wobei sich die Ausstülpung in Richtung der einen Seite oder der anderen Seite der dehnbaren Lage (40) in einer Dickenrichtung der dehnbaren Lage ausstülpt.

11. Dehnbare Lage (40) nach Anspruch 1 oder 2, wobei
jede der Verbindungsgruppen (50G) Folgendes einschließt:
ein Verbindungsteil des oberen Endes (50up), das sich auf das Verbindungsteil (50) an einem oberen vertikalen Ende von jeder der Verbindungsteilgruppen (50G) bezieht;
ein Verbindungsteil des unteren Endes (50dw), das sich auf das Verbindungsteil (50) an einem unteren vertikalen Ende von jeder der Verbindungsteilgruppen (50G) bezieht, und
eine vertikale Länge des Verbindungsteils des oberen Endes (50up) und eine vertikale Länge des Verbindungsteils des unteren Endes (50dw) jeweils länger sind als eine vertikale Länge von jedem der Verbindungsteile (50), außer dem Verbindungsteil des oberen Endes (50up) und dem Verbindungsteil des unteren Endes (50dw), in jeder der Verbindungsteilgruppen.

12. Unterhosen-förmiger absorbierender Artikel (1), der eine vertikale Richtung, eine laterale Richtung und eine Von-Hinten-Richtung aufweist, die einander schneiden, wobei der Unterhosen-förmige absorbierende Artikel (1) Folgendes umfasst:
die dehnbare Lage (40) nach Anspruch 1 oder 2;
einen absorbierenden Hauptkörper (10); und
ein Paar von Taillenteilen (20, 30),
wobei die dehnbare Lage (40) in mindestens einem des Paars von Taillenteilen (20, 30) bereitgestellt ist,
sich die vertikale Richtung der dehnbaren Lage (40) entlang der vertikalen Richtung des Unterhosen-förmigen absorbierenden Artikels (1) erstreckt,
sich die laterale Richtung der dehnbaren Lage (40) entlang der lateralen Richtung des Unterhosen-förmigen absorbierenden Artikels (1) erstreckt,
sich eine Dickenrichtung der dehnbaren Lage (40) entlang der Von-Hinten-Richtung des Unterhosen-förmigen absorbierenden Artikels (1) erstreckt,
das Paar von Taillenteilen (20, 30) durch ein Paar von Seitenverbindungsteilen (SS) an zwei lateralen Seiten des Paars von Taillenteilen (20, 30) verbunden ist,
mindestens einer des Paars von Taillenteilen (20, 30) einen geschichteten Teil oder einen gefalteten Teil (22r, 32r) einschließt,
wobei der geschichtete Teil ein Teil ist, in dem das Paar von Lagen an einer anderen Lage, außer dem Paar von Lagen, über einen Haftungsteil (70) haftet, sodass das Paar von Lagen und die andere Lage in Bezug auf die Vorn-Hinten-Richtung miteinander überlappen,
der gefaltete Teil (22r, 32r) ein Teil ist, in dem mindestens eine des Paars von Lagen in Richtung einer inneren Oberfläche des mindestens einen des Paars von Taillenteilen (20, 30) gefaltet ist und an der inneren Oberfläche des mindestens einen der Taillenteile (20, 30) über den Haftungsteil (70) haftet,
wobei der Unterhosen-förmige absorbierende Artikel (1) einen Teil einschließt, in dem der Nicht-Anordnungsbereich mit einem Nicht-Haftungsteil, außer dem Haftungsteil (70), in dem geschichteten Teil oder einem Nicht-Haftungsteil, außer dem Haftungsteil (70), in dem gefalteten Teil (22r, 32r) in einer Draufsicht überlappt.

## Revendications

1. Feuille extensible (40) ayant une direction verticale et une direction latérale qui se croisent l'une par rapport à l'autre,
la feuille extensible (40) comportant :
une pluralité d'éléments élastiques qui sont situés entre une paire de feuilles assemblées par une pluralité de parties d'assemblage (50),
les éléments de la pluralité d'éléments élastiques (23) étant agencés selon des intervalles dans la direction verticale,
les éléments de la pluralité d'éléments élastiques étant en mesure de s'étirer et de se contracter dans la direction latérale ; et
un groupe de parties d'assemblage (50G) qui comprend au moins une paire de parties d'assemblage (50) prenant en sandwich chacun des éléments élastiques,
une pluralité de groupes de parties d'assemblage (50G) constituant une première rangée de parties d'assemblage, une deuxième rangée de parties d'assemblage et une troisième rangée de parties d'assemblage qui sont agencées dans la direction verticale,
dans un état dans lequel la feuille extensible (40) est étirée dans la direction latérale,
dans chacun des groupes de parties d'assemblage (50G), une longueur verticale entre les parties d'assemblage (50) qui sont agencées de manière adjacente dans la direction verticale étant plus courte qu'une longueur latérale de chacune des parties d'assemblage (50),
une région de non-agencement (Np) étant située entre les groupes de parties d'assemblage (50G) qui sont agencées de manière adjacente dans la direction verticale,
la région de non-agencement (Np) étant une région dans laquelle les groupes de parties d'assemblage (50G) ne sont pas agencés,
une longueur verticale de la région de non-agencement (Np) étant plus longue que la longueur verticale entre la paire de parties d'assemblage (50),
la première rangée de parties d'assemblage, la deuxième rangée de parties d'assemblage et la troisième rangée de parties d'assemblage étant agencées de manière adjacente dans la direction latérale,
dans une certaine position dans la direction verticale, l'une des parties d'assemblage (50) dans la première rangée de parties d'assemblage, l'une des parties d'assemblage (50) dans la deuxième rangée de parties d'assemblage et l'une des parties d'assemblage (50) dans la troisième rangée de parties d'assemblage se chevauchant les unes les autres,
dans une autre certaine position dans la direction verticale, la région de non-agencement (Np) dans la première rangée de parties d'assemblage, la région de non-agencement (Np) dans la deuxième rangée de parties d'assemblage et la région de non-agencement (Np) dans la troisième rangée de parties d'assemblage se chevauchant les unes les autres.

2. Feuille extensible (40) selon la revendication 1, dans laquelle
dans un état dans lequel la feuille extensible est étirée dans la direction latérale,
une longueur verticale de chacun des groupes de parties d'assemblage (50G) est plus longue que la longueur latérale de chacun des groupes de parties d'assemblage (50G).

3. Feuille extensible (40) selon la revendication 1 ou la revendication 2, dans laquelle
dans un état dans lequel la feuille extensible (40) est étirée dans la direction latérale,
parmi la région de non-agencement (Np) dans la première rangée de parties d'assemblage, la région de non-agencement (Np) dans la deuxième rangée de parties d'assemblage et la région de non-agencement (Np) dans la troisième rangée de parties d'assemblage qui se chevauchent les unes les autres dans ladite une autre certaine position,
deux régions de non-agencement (Np) comprennent :
une région de chevauchement dans laquelle les deux régions de non-agencement se chevauchent l'une l'autre par rapport à la direction verticale ; et
une région de non-chevauchement dans laquelle les deux régions de non-agencement ne se chevauchent pas l'une l'autre par rapport à la direction verticale, et
une longueur verticale de la région de chevauchement est plus longue qu'une longueur verticale de la région de non-chevauchement.

4. Feuille extensible (40) selon la revendication 1 ou la revendication 2, dans laquelle
dans un état dans lequel la feuille extensible (40) est étirée dans la direction latérale,
la longueur verticale de la région de non-agencement (Np) est plus longue que la longueur verticale de chacun des groupes de parties d'assemblage.

5. Feuille extensible (40) selon la revendication 4, dans laquelle
la longueur verticale de la région de non-agencement (Np) est trois fois plus longue ou moins que la longueur verticale de chacun des groupes de parties d'assemblage (50G).

6. Feuille extensible (40) selon la revendication 1 ou la revendication 2, dans laquelle
dans un état dans lequel la feuille extensible (40) est étirée dans la direction latérale,
la longueur verticale de la région de non-agencement (Np) est inférieure à la longueur verticale de chacun des groupes de parties d'assemblage (50G).

7. Feuille extensible (40) selon la revendication 1 ou la revendication 2, dans laquelle
dans un état dans lequel la feuille extensible (40) est étirée dans la direction latérale,
une région de non-agencement entre des rangées est située entre les groupes de parties d'assemblage (50G) qui sont agencés de manière adjacente dans la direction latérale,
la région de non-agencement entre des rangées étant une région dans laquelle les groupes de parties d'assemblage (50G) ne sont pas agencés, et
une longueur latérale de la région de non-agencement entre des rangées est plus longue que la longueur verticale de chacun des groupes de parties d'assemblage (50G).

8. Feuille extensible (40) selon la revendication 7, dans laquelle
la longueur latérale de la région de non-agencement entre des rangées est trois fois plus longue ou moins que la longueur verticale de chacun des groupes de parties d'assemblage (50G).

9. Feuille extensible (40) selon la revendication 1 ou la revendication 2, dans laquelle
une partie d'assemblage auxiliaire (50) qui assemble les feuilles de la paire de feuilles ensemble est mise en œuvre dans une partie de la région de non-agencement,
la partie d'assemblage auxiliaire (50) étant différente des parties d'assemblage (50G).

10. Feuille extensible (40) selon la revendication 1 ou la revendication 2, dans laquelle
dans un état dans lequel les éléments élastiques (23) se contractent,
la région de non-agencement (Np) dans la première rangée de parties d'assemblage, la région de non-agencement dans la deuxième rangée de parties d'assemblage et la région de non-agencement (Np) dans la troisième rangée de parties d'assemblage qui se chevauchent les unes les autres dans ladite une autre position dans la direction verticale ont chacune une partie saillante,
la partie saillante faisant saillie vers un côté ou un autre côté de la feuille extensible (40) dans la direction allant dans le sens de l'épaisseur de la feuille extensible.

11. Feuille extensible (40) selon la revendication 1 ou la revendication 2, dans laquelle
chacun des groupes d'assemblage (50G) comprend :
une partie d'assemblage d'extrémité supérieure (50up) faisant référence à la partie d'assemblage (50) sur une extrémité verticale supérieure de chacun des groupes de parties d'assemblage (50G) ;
une partie d'assemblage d'extrémité inférieure (50dw) faisant référence à la partie d'assemblage (50) sur une extrémité verticale inférieure de chacun des groupes de parties d'assemblage (50G), et
une longueur verticale de la partie d'assemblage d'extrémité supérieure (50up) et une longueur verticale de la partie d'assemblage d'extrémité inférieure (50dw) sont chacune plus longues qu'une longueur verticale de chacune des parties d'assemblage (50) autres que la partie d'assemblage d'extrémité supérieure (50up) et la partie d'assemblage d'extrémité inférieure (50dw) dans chacun des groupes de parties d'assemblage.

12. Article absorbant en forme de culotte (1) ayant une direction verticale, une direction latérale et une direction allant d'avant en arrière qui se croisent les unes par rapport aux autres,
l'article absorbant en forme de culotte (1) comportant :
la feuille extensible (40) selon la revendication 1 ou la revendication 2 ;
un corps principal absorbant (10) ; et
une paire de parties au niveau de la taille (20, 30),
la feuille extensible (40) étant mise en œuvre dans au moins l'une de la paire de parties au niveau de la taille (20, 30),
la direction verticale de la feuille extensible (40) s'étendant le long de la direction verticale de l'article absorbant en forme de culotte (1),
la direction latérale de la feuille extensible (40) s'étendant le long de la direction latérale de l'article absorbant en forme de culotte (1),
une direction allant dans le sens de l'épaisseur de la feuille extensible (40) s'étendant le long de la direction allant d'avant en arrière de l'article absorbant en forme de culotte (1),
les parties de la paire de parties au niveau de la taille (20, 30) étant assemblées ensemble par les parties d'une paire de parties d'assemblage latérales (SS), sur deux côtés latéraux de la paire de parties au niveau de la taille (20, 30),
au moins l'une de la paire de parties au niveau de la taille (20, 30) comprenant une partie empilée ou une partie pliée (22r, 32r),
la partie empilée étant une partie dans laquelle la paire de feuilles adhère à une autre feuille autre que la paire de feuilles par le biais d'une partie adhésive (70) de telle sorte que la paire de feuilles et ladite une autre feuille se chevauchent les unes les autres par rapport à la direction allant d'avant en arrière,
la partie pliée (22r, 32r) étant une partie dans laquelle au moins l'une de la paire de feuilles est pliée vers une surface intérieure de ladite au moins l'une de la paire de parties au niveau de la taille (20, 30) et adhère à la surface intérieure de ladite au moins l'une des parties au niveau de la taille (20, 30) par le biais de la partie adhésive (70),
l'article absorbant en forme de culotte (1) comprenant une partie dans laquelle la région de non-agencement est chevauchée par une partie non adhésive autre que la partie adhésive (70) dans la partie empilée ou une partie non adhésive autre que la partie adhésive (70) dans la partie pliée (22r, 32r), dans une vue en plan.
